# FASCICULE DE BREVET EUROPEEN

(11) **EP 3 285 719 B1**
(45) Date de publication et mention de la délivrance du brevet: **28.08.2019**
(21) Numéro de dépôt: 16729304.2
(22) Date de dépôt: 08.04.2016
(51) Int. Cl.: A61K 8/26, A61K 8/33, A61K 8/34, A61K 8/37, A61Q 5/00, A61K 8/60, A61Q 19/00, A61K 8/81, A61K 8/04, A61K 8/06, A61K 8/39, A61K 8/86, C09D 133/06

(54) **NOUVELLES ÉMULSIONS COSMÉTIQUES SE PRÉSENTANT SOUS LA FORME DE MOUSSE, PROCÉDÉ POUR LEUR OBTENTION ET LEUR UTILISATION EN COSMÉTIQUE**
NEUARTIGE KOSMETISCHE EMULSIONEN IN SCHAUMFORM, VERFAHREN ZUR HERSTELLUNG DAVON UND KOSMETISCHE VERWENDUNG DAVON
NOVEL COSMETIC EMULSIONS PROVIDED IN FOAM FORM, METHOD FOR OBTAINING SAME AND COSMETIC USE THEREOF

(30) Priorité: 20.04.2015 FR 1553511; 17.11.2015 FR 1561066
(43) Date de publication de la demande: 28.02.2018
(73) Titulaire: Société d'Exploitation de Produits pour les Industries Chimiques SEPPIC, 75321 Paris Cedex 7 (FR)
(72) Inventeur: TAILLEBOIS, Cécile, 81990 Salies (FR); SIGURANI, Séverine, 31200 TOULOUSE (FR); SOUYRI, Benoît, 81100 Castres (FR)
(74) Mandataire: Conan, Philippe Claude
(86) Numéro de dépôt international: PCT/FR2016/050820
(87) Numéro de publication internationale: WO 2016/170246

(56) Documents cités:
- FR-A1- 2 915 896
- FR-A1- 2 979 234
- FR-A1- 2 983 722
- FR-A1- 3 004 454
- US-A1- 2004 197 279

## Description

La présente invention a pour objet de nouvelles émulsions se présentant sous la forme de mousses, leur procédé de préparation, leur utilisation en tant que compositions topiques cosmétiques destinées au soin et au nettoyage de la peau, des cheveux, du cuir chevelu, des muqueuses et des ongles, ainsi qu'au maquillage de la peau, des ongles et des cheveux.

Les compositions cosmétiques peuvent se présenter sous une forme monophasique, comme par exemple les solutions aqueuses ou hydro-alcooliques ou hydro-glycoliques, dans lesquelles l'ensemble des constituants de la composition cosmétique est soluble dans un solvant. Cependant, les compositions cosmétiques les plus couramment utilisées se présentent sous une forme multiphasique et plus particulièrement sous une forme dite « dispersée » pour laquelle une phase de nature liquide ou gazeuse est maintenue dispersée de façon stable dans une phase continue liquide.

Parmi les formes dispersées couramment utilisées par les industries de la cosmétique et de la pharmacie, on peut citer les compositions se présentant sous la forme de mousses.

Les mousses sont des structures constituées par un ensemble de cellules gazeuses, sphériques ou polyédriques, séparées par des lamelles minces de liquides, formées par la juxtaposition de bulles que génère un gaz dispersé dans un liquide. Si les lamelles sont perturbées ou si elles refluent dans les cellules gazeuses, on observe alors une coalescence des cellules gazeuses qui aboutit à une déstabilisation de la structure globale et *in fine* à la séparation de la phase liquide et de la phase gazeuse, et donc à la disparition de la forme mousse. Les mousses sont également des formes galéniques thermodynamiquement instables et le défi du chercheur réside alors à trouver des moyens de renforcer la stabilité de la mousse pour éviter une disparition trop rapide de cette forme physique. Ainsi, une composition sous forme physique de mousse peut être stabilisée dans le temps par l'emploi d'un système tensioactif adapté et/ou d'un agent chimique auxiliaire choisi parmi les éléments compris dans le groupe constitué par les amines grasses, les alcools gras, les alcanols amides gras ou les amino-oxydes tertiaires, tel que décrit dans la demande internationale de brevet publiée sous le numéro WO 93/22538 A1.

Les mousses sont généralement préparées à partir de compositions liquides par mélange d'un gaz, comme par exemple l'air ou l'azote ou le dioxyde de carbone, à condition que le liquide dans lequel est dispersé le gaz comprenne au moins un agent tensioactif adapté, qui présente à la fois des propriétés d'activité inter-faciale et la capacité d'organiser le liquide à l'interface sous la forme d'un film. Le mélange du gaz dans lesdites compositions liquides, comprenant un agent tensioactif adapté, destiné à atteindre la forme d'une mousse, s'effectue généralement par barbotage dudit gaz mais peut également être réalisé par une agitation à très forte vitesse desdites compositions sous atmosphère du gaz que l'on cherche à disperser. Le procédé de formation de compositions cosmétiques se présentant sous la forme d'une mousse préféré est celui introduisant le gaz par barbotage car les autres procédés demandent un apport énergétique très élevé, voire l'utilisation de mobile d'agitation non conventionnel et fortement couteux à l'échelle industrielle.

De telles compositions cosmétiques, comprenant des agents tensioactifs adaptés et se présentant sous la forme de mousse, se caractérisent par une grande surface spécifique interne induisant une capacité d'adsorption élevée, rendant ainsi de telles compositions sous forme de mousse particulièrement adaptées pour une utilisation de nettoyage de la peau, des cheveux, du cuir chevelu. Les compositions cosmétiques se présentant sous la forme d'une mousse présentent aussi l'avantage de permettre une fine répartition des ingrédients actifs, compris dans ladite mousse, sur la peau.

Un inconvénient des compositions cosmétiques se présentant sous la forme de mousse, réside dans l'emploi d'une quantité importante d'agents tensioactifs, souvent supérieure à une proportion massique de 5%, qui augmente alors le risque d'une moindre tolérance cutanée par les consommateurs. Mais l'inconvénient majeur des compositions cosmétiques se présentant sous la forme de mousse, consiste en la faible stabilité de tels systèmes, qui perdent leur aspect de mousse dans les 24 heures suivant leur formation, ce qui constitue une durée de stabilité non compatible avec les exigences de durées de stabilité de compositions cosmétiques.

Cet inconvénient est contourné en fournissant au consommateur la composition cosmétique sous forme liquide dans un contenant dans lequel se trouve également un gaz liquéfié sous pression servant de gaz propulseur. En ouvrant une soupape de pression, le mélange de composition liquide et de gaz propulseur s'échappe du contenant à travers d'une buse et, le gaz propulseur s'évaporant, laisse une mousse. Le consommateur peut ainsi générer la forme mousse de la composition cosmétique avant son application sur la peau.

Cependant, cette solution couramment utilisée par les consommateurs concerne essentiellement les compositions cosmétiques à base de tensioactifs adaptés, et qui repose sur un schéma de formulation simple, à base de quelques ingrédients, ne permettant pas d'atteindre des compositions sous forme de mousse plus complexes mais adaptées aux besoins et exigences actuels des consommateurs.

Dans cette optique, des solutions visant à présenter des émulsions sous la forme de mousses ont été développées dans l'état de la technique. Les émulsions sont les formes galéniques les plus couramment utilisées par l'industrie cosmétique et par l'industrie pharmaceutique, pour apporter de multiples propriétés aux consommateurs. L'introduction d'une phase gazeuse à disperser dans une émulsion doit être réalisée de telle sorte à ne pas déstabiliser ladite émulsion. Par conséquent, pour des émulsions utilisant un système tensioactif émulsionnant conventionnel, soit la quantité de gaz effectivement dispersée est trop faible pour obtenir les avantages d'une mousse soit la quantité de gaz effectivement dispersée est trop importante et génère une instabilité au cours du stockage.

Pour résoudre ces problèmes, l'art antérieur fait état de systèmes émulsionnants ternaires (S_{T}), comprenant :
(i) au moins un émulsionnant (A) choisi parmi les acides gras, saturés ou insaturés, linéaires ou ramifiés, totalement ou partiellement salifiés, comportant de 10 à 40 atomes de carbone,
(ii) au moins un émulsionnant (B) choisi parmi les esters polyéthoxylés d'acides gras, comportant de 10 à 40 atomes de carbone et ayant un degré d'éthoxylation compris entre 5 et 100,
(iii) au moins un émulsionnant (C) choisi parmi les alcools gras saturés ou insaturés, linéaires ou ramifiés, comportant de 10 à 40 atomes de carbone.

La demande de brevet américain publiée sous le numéro US 2004/0161437 A1 décrit des compositions cosmétiques se présentant sous la forme d'une émulsion, comprenant une phase aqueuse, au moins une phase lipidique, une quantité de 1% à 90% en volume de gaz, une quantité massique de 0,1% à 10% d'au moins un gélifiant inorganique, une quantité massique de 0,1% à 10% d'au moins un composé hydrophobe particulaire, et le système émulsionnant (S_{T}) décrit ci-dessus.

La demande de brevet américain publiée sous le numéro US 2004/0197279 A1 décrit des compositions cosmétiques se présentant sous la forme d'une émulsion, comprenant une phase aqueuse, au moins une phase lipidique, une quantité de 1% à 90% en volume de gaz, une quantité massique de 0,1% à 10% d'au moins un gélifiant inorganique, et au moins un hydrocolloïde organique, et le système émulsionnant (S_{T}) décrit ci-dessus.

La demande de brevet américain publiée sous le numéro US 2009/0017079 A1 décrit des émulsions se présentant sous forme de mousses, stables, dont la phase continue comprend au moins un système épaississant et dont la phase discontinue comprend un agent structurant ; des telles émulsions étant préparées par mélange des différents phases à une température supérieure à la température critique du système épaississant.

Les émulsions cosmétiques se présentant sous la forme de mousses, telles que décrites dans l'état de la technique, sont préparées par la mise en oeuvre de procédés impliquant une maîtrise technique élevée, lesdits procédés comprenant au moins une étape de barbotage du gaz dans l'émulsion formée au préalable à une pression déterminée, généralement comprise entre 1 et 2,5 bars, à une température maîtrisée.

De façon à obtenir des émulsions cosmétiques se présentant sous la forme de mousses stables, fonctionnelles, adaptées aux besoins des industries cosmétiques, la demanderesse a donc développé de nouvelles émulsions cosmétiques de type huile-dans-eau se présentant sous la forme de mousses et obtenues selon un procédé de préparation ne mettant pas en oeuvre d'étape de barbotage d'un gaz dans ladite émulsion.

C'est pourquoi selon un premier aspect, l'invention a pour objet une émulsion (E₁) de type huile-dans-eau se présentant sous la forme d'une mousse, caractérisée en ce qu'elle comprend pour 100% de sa masse :
a) - De 27% à 89,9% massique, de 27% à 84,5% massique, plus particulièrement de 42% à 89,5% massique et encore plus particulièrement de 52,5% à 86,5% massique d'une phase aqueuse (A₁) cosmétiquement acceptable ;
b) - De 0,1% à 3% massique, plus particulièrement de 0,5% à 3% massique et encore plus particulièrement de 0,5% à 2,5% massique d'un polyélectrolyte anionique réticulé (P) issu de la polymérisation, en présence d'au moins un agent de réticulation, de l'acide 2-méthyl 2-[(1-oxo 2-propènyl) amino] 1-propanesulfonique partiellement ou totalement salifié, avec au moins un monomère neutre choisi parmi l'acrylamide, l'acrylate de (2-hydroxy éthyle), les N,N-dialkyl acrylamides, dans lesquels chacun des groupes alkyle comportent entre un et quatre atomes de carbones, et au moins un monomère de formule (I) : 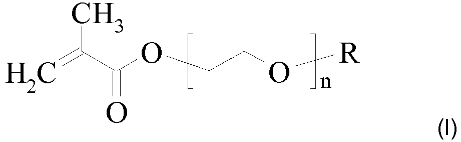 dans laquelle R représente un radical alkyle linéaire ou ramifié comportant de huit à vingt atomes de carbone et n représente un nombre entier supérieur ou égal à un et inférieur ou égal à vingt ;
c) - De 5% à 20% massique, plus particulièrement de 5% à 15% massique et encore plus particulièrement de 8% à 15% massique d'au moins un agent épaississant inorganique (Ei) choisi parmi les phyllosilicates naturels ou synthétiques, modifiés ou non modifiés ;
d) - De 5% à 50% massique, plus particulièrement de 5 % à 40% massique et encore plus particulièrement de 5% à 30% massique d'une phase grasse (A₂) comprenant pour 100% de sa masse :
   d₁) - De 0,2% à 25% massique, notamment de 0,2 à 20% massique, plus particulièrement de 0,25% à 12,5% massique et encore plus particulièrement de 0,25% à 10% massique d'un système émulsionnant (S) comprenant un ou plusieurs tensioactifs émulsionnants sélectionnés parmi les compositions d'alkylpolyglycosides ; les compositions d'alkylpolyglycosides et d'alcools gras; les acides gras polyéthoxylés comportant de 10 à 40 atomes de carbone et ayant un degré d'éthoxylation compris entre 5 et 100 ; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone ; les alcools gras polyéthoxylés comportant de 10 à 40 atomes de carbone et ayant un degré d'éthoxylation compris entre 2 et 100 ; les compositions d'alcools gras polyéthoxylés comportant de 10 à 40 atomes de carbone et ayant un degré d'éthoxylation compris entre 2 et 100 et d'alcools gras comportant de 12 à 40 atomes de carbone ; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone et d'alcools gras comportant de 12 à 40 atomes de carbone ; et
   d₂) - De 75% à 99,8% massique, notamment de 80% à 99,8% massique, plus particulièrement de 87,5% à 99,75% et encore plus particulièrement de 90% à 99,75% massique d'au moins une huile et optionnellement d'au moins une cire ;
      ladite émulsion (E₁) étant en outre caractérisée en ce que le rapport massique entre l'agent épaississant inorganique (Ei) et le polyélectrolyte anionique réticulé (P) est supérieur ou égal à 6/1 et inférieur ou égal à 20/1, plus particulièrement supérieur ou égal à 8/1 et inférieur ou égal à 15/1 et encore plus particulièrement supérieur ou égal à 10/1 et inférieur ou égal à 15/1.

L'expression « cosmétiquement acceptable » utilisée dans la définition de la phase aqueuse (A₁) présente dans l'émulsion (E₁) objet de la présente invention, signifie selon la directive du Conseil de la Communauté Economique Européenne N°76/768/CEE du 27 juillet 1976 modifiée par la directive N°93/35/CEE du 14 juin 1993, qu'elle comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Selon un aspect particulier, l'émulsion (E₁) telle que définie ci-dessus comprend pour 100% de sa masse :
a) - De 52,5% à 80% massique de ladite phase aqueuse (A₁).

Par huile, on désigne, dans la définition de l'émulsion (E₁) objet de la présente invention, un composé et/ou un mélange de composés insoluble dans l'eau, et liquide à 25°C, et plus particulièrement :
- Les huiles minérales telles que l'huile de paraffine, l'huile de vaseline, les isoparaffines ou les huiles blanches minérales ;
- Les huiles d'origine animale, telles que le squalène ou le squalane ;
- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- Les huiles végétales éthoxylées ;
- Les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées et
- Les huiles de silicone comme les diméthylpolysiloxanes, les méthylphényl-polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Selon un autre aspect particulier, l'émulsion (E₁) telle que définie ci-dessus comprend pour 100% de sa masse :
d) - De 15 % à 40% massique et plus particulièrement de 15% à 30% massique, de ladite phase grasse (A₂).

Lorsque l'émulsion (E₁) telle que définie ci-dessus comprend un cire, cette dernière est plus particulièrement choisie parmi la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline; l'ozokérite; la cire de polyéthylène; les cires de silicone ; les cires végétales ; les alcools gras et les acides gras solides à température ambiante; les glycérides solides à température ambiante.

Par polyélectrolyte anionique réticulé (P), on désigne, dans la définition de l'émulsion (E₁) objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Selon un autre aspect particulier, l'émulsion (E₁) telle que définie précédemment, comprend pour 100% de sa masse :
a) - De 27% à 84,5% massique de ladite phase aqueuse (A₁) ;
b) - De 0,5% à 3% massique dudit polyélectrolyte anionique réticulé (P).

Par partiellement salifié ou totalement salifié, on signifie dans la définition du polyélectrolyte anionique réticulé (P) présent de l'émulsion (E₁) telle que définie ci-dessus, que ledit acide 2-méthyl 2-[(1-oxo 2-propènyl) amino] 1-propanesulfonique est partiellement ou totalement salifié, notamment sous forme de sel de métal alcalin, par exemple sous forme de sel de sodium ou de sel de potassium, ou sous forme de sel d'ammonium.

Par monomère neutre choisi parmi les N,N-dialkyl acrylamides, dans lesquels chacun des groupes alkyle comportent entre un et quatre atomes de carbones, on désigne, dans la définition de l'émulsion (E₁) objet de la présente invention, plus particulièrement le N,N-diméthyl acrylamide, le N,N-diéthyl acrylamide, le N,N-dipropyl acrylamide, ou le N,N-di-isopropyl acrylamide.

Selon un aspect particulier de la présente invention, dans l'émulsion (E₁) telle que définie ci-dessus, ledit polyélectrolyte anionique réticulé (P) comporte pour 100% molaire de ses monomères constitutifs, de 5% molaire à 95% molaire, plus particulièrement de 10% molaire à 90% molaire, et tout particulièrement de 20% molaire à 80% molaire d'unités monomériques issues de l'acide 2-méthyl 2-[(1-oxo 2-propènyl) amino] 1-propanesulfonique partiellement ou totalement salifié,

Selon un autre aspect particulier de la présente invention, dans l'émulsion (E₁) telle que définie ci-dessus, ledit polyélectrolyte anionique réticulé (P) comporte pour 100% molaire de ses monomères constitutifs, de 4,9% molaire à 90% molaire, plus particulièrement de 9,5% molaire à 85% molaire, et tout particulièrement de 15% molaire à 75% molaire d'unités monomériques issues d'au moins un monomère neutre choisi parmi l'acrylamide, l'acrylate de (2-hydroxy éthyle), les N,N-dialkyl acrylamides, dans lesquels chacun des groupes alkyle comportent entre un et quatre atomes de carbones, dans lesquels chacun des groupes alkyle comportent entre un et quatre atomes de carbones.

Selon un autre aspect particulier de la présente invention, dans l'émulsion (E₁) objet de la présente invention, ledit polyélectrolyte anionique réticulé (P) tel que défini précédemment comporte pour 100% molaire de ses monomères constitutifs, de 0,1% molaire à 10% molaire et plus particulièrement de 0,5% molaire à 5% molaire, d'unités monomériques issues du monomère de formule (I).

L'invention a plus particulièrement pour objet une émulsion (E₁) telle que définie précédemment, caractérisée en ce que ledit polyélectrolyte anionique réticulé (P) comporte, pour 100% molaire de ses monomères constitutifs :
- De 20% molaire à 80% molaire d'unités monomériques issues dudit acide 2-méthyl 2-[(1-oxo 2-propènyl) amino] 1-propanesulfonique partiellement ou totalement salifiée ;
- De 15% molaire à 75% molaire d'unités monomériques issues d'au moins un monomère neutre choisi parmi l'acrylamide, l'acrylate de (2-hydroxy éthyle), les N,N-dialkyl acrylamides, dans lesquels chacun des groupes alkyle comportent entre un et quatre atomes de carbones,
- De 0,5% à 5% molaire d'unités monomériques issues dudit monomère de formule (I) telle que définie précédemment.

Dans la formule (I) du monomère présent dans ledit polyélectrolyte anionique réticulé (P) compris dans l'émulsion (E₁) objet de la présente invention, par radical alkyle linéaire ou ramifié comportant de huit à vingt atomes de carbone, on désigne plus particulièrement pour R :
- ou bien un radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle ou eicosyle ;
- ou bien un radical dérivé des alcools de Guerbet, qui sont des 1-alcanols ramifiés répondant à la formule générale :

   CH₃-(CH₂)ₚ-CH[CH₃-(CH₂)ₚ₋₂]-CH₂OH,

   dans laquelle p représente un nombre entier compris entre 2 et 9, tels que, par exemple, les radicaux 2-éthyl hexyle, 2-propyl heptyle, 2-butyl octyle, 2-pentyl nonyle, 2-hexyl décyle ou 2-octyl dodécyle ;
- ou bien un radical dérivé des isoalcanols répondant à la formule générale :

   CH₃-CH(CH₃)-(CH₂)ₘ-CH₂OH,
dans laquelle m représente un nombre entier compris entre 2 et 16, tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle.

Selon un autre aspect particulier, l'émulsion (E₁) telle que définie précédemment, est caractérisée en ce que ledit monomère neutre est choisi parmi l'acrylamide, l'acrylate de (2-hydroxy éthyle) ou le N,N-diméthyl acrylamide.

Selon un autre aspect encore plus particulier, l'émulsion (E₁) telle que définie précédemment, est caractérisée en ce que ledit monomère neutre est le N,N-diméthyl acrylamide.

Selon un autre aspect particulier, dans la formule (I) telle que définie précédemment, R représente un radical alkyle comportant de 12 à 18 atomes de carbone.

Selon un aspect encore plus particulier, l'invention a pour objet une émulsion (E₁) telle que définie précédemment, caractérisée en ce que dans la formule (I) telle que définie précédemment, R représente un radical alkyle choisi parmi le radical dodécyle, tridécyle, tétradécyle, hexadécyle, octadécyle.

Selon un autre aspect particulier, l'invention a pour objet une composition (E₁) telle que définie précédemment, caractérisée en ce que dans la formule (I) telle que définie précédemment, n représente un nombre entier supérieur ou égal à 3 et inférieur ou égal à 20.

Selon un aspect encore plus particulier, l'émulsion (E₁) telle que définie précédemment, est caractérisée en ce que ledit monomère de formule (I) est le méthacrylate de lauryle tétraéthoxylé.

Selon un autre aspect particulier, l'invention a pour objet une émulsion (E₁) telle que définie précédemment, caractérisée en ce que ledit polyélectrolyte anionique réticulé (P) tel que défini précédemment est réticulé avec un composé diéthylénique ou polyéthylènique dans la proportion molaire exprimée par rapport aux monomères mis en oeuvre, est de 0,005% à 1% molaire, plus particulièrement de 0,01% à 0,5% molaire et tout particulièrement de 0,01% à 0,25% molaire. L'agent de réticulation est plus particulièrement choisi parmi le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Dans le procédé de préparation du polyélectrolyte anionique réticulé (P) mis en oeuvre dans l'émulsion (E₁) objet de la présente invention, on peut également mettre en oeuvre lors de la réaction de polymérisation, divers additifs, tels que des agents complexants, des agents de transfert ou des agents limiteurs de chaîne. Les agents de transfert ou limiteurs de chaîne sont plus particulièrement choisi parmi le groupe constitué par l'hypophosphite de sodium, des alcools de faibles poids moléculaires par exemple le méthanol, l'éthanol, le 1-propanol, l'isopropanol, le butanol, des thiols, par exemple le 2-mercapto éthanol, des agents de transfert comprenant une fonction sulfate, par exemple le methallylsulfonate de sodium, ou des mélanges desdits agents de transfert. Les agents de transfert ou limiteurs de chaînes sont plus particulièrement utilisés dans des proportions molaires, exprimées par rapport au nombre total de moles de monomères mis en oeuvre, de 0,001% à 1% molaire, plus particulièrement de 0,001% à 0,5% molaire, et tout particulièrement de 0,001% à 0,1% molaire.

Selon un aspect particulier, l'invention a pour objet une émulsion (E₁) telle que définie précédemment, caractérisée en ce que ledit polyélectrolyte anionique réticulé (P) tel que défini précédemment est choisi parmi les terpolymères de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate ou parmi les terpolymères de l'acide 2-méthyl 2- [(1-oxo 2-propènyl) amino]1-propanesulfonique partiellement salifié sous forme de sel d'ammonium, du N,N-diméthyl acrylamide et du méthacrylate de stéaryle eicosaéthoxylé, réticulé au triméthylol propanetriacrylate.

Selon un aspect plus particulier, l'invention a pour objet une émulsion (E₁) telle que définie précédemment, caractérisée en ce que ledit polyélectrolyte anionique réticulé (P) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

Selon un autre aspect plus particulier, l'invention a pour objet une émulsion (E₁) telle que définie précédemment, caractérisée en ce que ledit polyélectrolyte anionique réticulé (P) comporte pour 100% molaire :
- De 60% molaire à 80% molaire d'unités monomériques issues de l'acide 2-méthyl 2-[(1-oxo 2-propènyl) amino] 1-propanesulfonique partiellement salifié sous forme d'ammonium,
- De 15% molaire à 39,5% molaire d'unités monomériques issues du N,N-diméthyl acrylamide, et
- De 0,5% molaire à 5% molaire d'unités monomériques issues du méthacrylate de lauryle tétraéthoxylé.

Selon un autre aspect particulier, l'émulsion (E₁) telle que définie ci-dessus comprend pour 100% de sa masse :
b) - De 0,5% à 2,5% massique dudit polyélectrolyte anionique réticulé (P).

Dans la définition de l'agent épaississant inorganique (Ei) présent dans l'émulsion (E₁) objet de la présente invention, on désigne par le terme « phyllosilicate » tout composé qui est un minéraux du groupe des silicates construit par empilement de couches tétraédrique (« T ») où les tétraèdres partagent trois sommets sur quatre (les oxygènes « basaux »), le quatrième sommet (l'oxygène « apical ») étant relié à une couche octaédrique (« O ») occupée par des cations différents comme par exemple les cations de l'aluminium, du magnésium, du fer, du titane, du lithium. L'union des couches T et des couches O forme des feuillets qui représentent l'unité de clivage des phyllosilicates.

Selon un autre aspect particulier, l'invention a pour objet une émulsion (E₁) telle que définie précédemment, caractérisée en ce que l'agent épaississant inorganique (Ei) est un phyllosilicate naturel ou synthétique, modifié ou non modifié, choisi parmi les silicates d'aluminium, les silicates de magnésium, ou les silicates d'aluminium et de magnésium.

Selon un aspect plus particulier, l'invention a pour objet une émulsion (E₁) telle que définie précédemment, caractérisée en ce que l'agent épaississant inorganique (Ei) est choisi parmi la kaolinite, la montmorillonite, l'illite, la beidellite, la saponite, la bentonite, l'hectorite, la vermiculite, la serpentine, la nacrite, l'amésite, la nontronite, la lizardite, la séricite, l'halloylsite, la muscovite, la paragonite, la damouzite, la glauconite ou la céladonite.

Selon un aspect encore plus particulier, l'invention a pour objet une émulsion (E₁) telle que définie précédemment, caractérisée en ce que l'épaississant inorganique (Ei) est choisi parmi la kaolinite, la montmorillonite, la saponite, la bentonite ou l'hectorite.

Selon un autre aspect particulier l'émulsion (E₁) telle que définie précédemment, comprend pour 100% de sa masse :
c) - De 10% à 15% massique dudit agent épaississant inorganique (Ei).

Selon un autre aspect particulier dans l'émulsion (E₁) telle que définie précédemment, le rapport massique entre ledit agent épaississant inorganique (Ei) et ledit polyélectrolyte anionique réticulé (P) est supérieur ou égal à 8/1.

Dans la définition de l'émulsion (E₁) objet de la présente invention, on désigne par composition d'alkylpolyglycosides comprise dans le système émulsionnant (S), une composition (C₁) représentée par la formule (II) :

R₁-O-(G)ₓ₋H (II)

dans laquelle x représente un nombre décimal compris entre 1,05 et 5, G représente le reste d'un sucre réducteur, et R₁ représente un radical aliphatique hydrocarboné, saturé ou insaturé, linéaire ou ramifié, éventuellement substitué avec un ou plusieurs groupe hydroxyle, comportant de 12 à 36 atomes de carbone, ladite composition (C₁) consistant en un mélange de composés représentés par les formules (I₁), (I₂), (I₃), (I₄) et (I₅) :

R₁-O-(G)₁-H (II₁)

R₁-O-(G)₂-H (II₂)

R₁-O-(G)₃-H (II₃)

R₁-O-(G)₄-H (II₄)

R₁-O-(G)₅-H (II₅)

dans les proportions molaires respectives a₁, a₂, a₃, a₄ et a₅, telles que :
- La somme a₁ + a₂ + a₃ + a₄ + a₅ est égale à 1 et que
- La somme a₁ + 2a₂ + 3a₃ + 4a₄ + 5a₅ est égale à x.

Par radical aliphatique hydrocarboné, saturé ou insaturé, linéaire ou ramifié, comportant de 12 à 36 atomes de carbone, éventuellement substitué avec un ou plusieurs groupes hydroxyle, on désigne pour le radical R₁ dans la formule (II) telle que définie ci-dessus :
- Les radicaux alkyle linéaires saturés, par exemple les radicaux n-dodécyle, n-tridécyle, n-tétradécyle, n-pentadécyle, n-hexadécyle, n-heptadécyle, n-octadécyle, n-nonadécyle, n-eicosyle, n-docosyle ;
- Les radicaux linéaires insaturés tels que les radicaux dodécènyle, tridécènyle, tétradécènyle, pentadécènyle, hexadécènyle, heptadécènyle, octadécènyle, nonadécènyle, eicosènyle, docosènyle, 4-dodécènyle, ou 5-dodécènyle ;
- Les radicaux aliphatiques saturés ou insaturés, linéaires ou ramifiés, comportant de 12 à 36 atomes de carbone substitués par un ou deux groupes hydroxy, tels que les radicaux hydroxydodécyle, hydroxytétradécyle, hydroxyhexadécyle, hydroxyoctadécyle, hydroxyeicosyle, hydroxydocosyle, par exemple le radical 12-hydroxy octadécyle.
- Les radicaux issus des isoalcanols de formule (1) :

   (CH₃)(CH₃)CH-(CH₂)ᵣ-CH₂-OH (1)

   dans laquelle r représente un nombre entier compris entre 8 et 20, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ; Les radicaux alkyles ramifiés, issus des alcools de Guerbet, de formule (2) :

   CH(CₛH₂ₛ₊₁)(CₜH₂ₜ₊₁)-CH₂-OH (2)

   dans laquelle t est un nombre entier compris entre 6 et 18, s est un nombre entier compris entre 4 et 18 et la somme s + t est supérieure ou égale à 10, et inférieure ou égale à 22, par exemple les radicaux 2-butyl octyle, 2-butyl décyle, 2-hexyl octyle, 2-hexyl décyle, 2-octyl décyle, 2-hexyl dodécyle, 2-octyl dodécyle, 2-décyl tétradécyle, 2-dodécyl hexadécyle, 2-tétradécyl octadécyle. Selon un aspect particulier, dans la définition de la formule (II) telle que définie ci-dessus, R₁ représente un radical aliphatique hydrocarboné, saturé ou insaturé, linéaire ou ramifié comportant de 12 à 24 atomes de carbone.

Par sucre réducteur, dans la définition de la formule (II) telle que définie ci-dessus, on désigne les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique (G)ₓ, peut se présenter sous toutes formes d'isoméries, qu'il s'agisse d'isomérie optique, d'isomérie géométrique ou d'isomérie de position ; elle peut aussi représenter un mélange d'isomères.

Dans la formule (II) telle que définie ci-dessus, le groupe R₁-O- est lié à G par le carbone anomérique du reste saccharide, de manière à former une fonction acétal.

Selon un aspect particulier dans la définition de la formule (II) telle que définie ci-dessus, G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose; et plus particulièrement G représente le reste d'un sucre réducteur choisi parmi les restes du glucose, du xylose et de l'arabinose.

Selon un aspect encore plus particulier, dans la définition de la formule (II) représentant la composition (C₁) comprise dans l'émulsion (E₁) objet de la présente invention, x représente un nombre décimal supérieur ou égal à 1,05 et inférieur ou égal à 2,5, plus particulièrement supérieur ou égal à 1,05 et inférieur ou égal à 2,0, et encore plus particulièrement supérieur ou égal à 1,25 et inférieur ou égal à 2,0.

Selon un aspect encore plus particulier, l'émulsion (E₁) telle que définie précédemment, est caractérisée en ce que, dans la définition dudit système émulsionnant (S), les compositions d'alkylpolyglycosides sont des compositions (C1) représentées par la formule (II) :

R1-O-(G)ₓ-H (II)

dans laquelle x représente un nombre décimal compris entre 1,05 et 2,5, G représente le reste d'un sucre réducteur, et R1 représente un radical aliphatique hydrocarboné, linéaire ou ramifié, saturé ou insaturé comportant de 12 à 36 atomes de carbone ; ladite composition (C1) consistant en un mélange de composés représentés par les formules (I₁), (I₂), (I₃), (I₄) et (I₅) :

R1-O-(G)₁-H (II₁)

R1-O-(G)₂-H (II₂)

R1-O-(G)₃-H (II₃)

R1-O-(G)₄-H (II₄)

R1-O-(G)₅-H (II₅)

dans les proportions molaires respectives a₁, a₂, a₃, a₄ et a₅, telles que :
- La somme a₁ + a₂ + a₃ + a₄ + a₅ est égale à 1 et que
- La somme a₁ + 2a₂ + 3a₃ + 4a₄ + 5a₅ est égale à x.

Selon un aspect encore plus particulier, dans la définition de la formule (II) telle que définie ci-dessus, R₁ représente le radical choisi parmi au moins un des éléments du groupe constitué par les radicaux n-dodécyle, n-tétradécyle, n-hexadécyle, n-octadécyle, n-eicosyle, n-docosyle, n-oléyle, n-linoléyle et n-linolényle , 2-hexyl décyle, 2-octyl décyle, 2-hexyl dodécyle, 2-octyl dodécyle, 2-décyl tétradécyle ; G représente le reste d'un sucre réducteur choisi parmi les restes du glucose et du xylose et x représente un nombre décimal supérieur ou égal à 1,05 et inférieur ou égal à 2,5.

Selon un aspect encore plus particulier, l'émulsion telle que définie précédemment est caractérisée en ce que dans la composition (C1) représentée dans la formule (II), x représente un nombre décimal compris entre 1,05 et 2,5, G représente le reste du glucose, et R1 représente un radical aliphatique hydrocarboné choisi parmi les radicaux n-dodécyle, n-tétradécyle, n-hexadécyle, n-octadécyle, n-eicosyle, n-docosyle , n-oléyle, n-linoléyle ou n-linolényle.

Selon un aspect encore plus particulier, l'invention a pour objet une émulsion (E₁) telle que définie précédemment caractérisée en ce que, dans la définition dudit système émulsionnant (S), les compositions d'alkylpolyglycosides et d'alcools gras sont des compositions (C2) comprenant pour 100% de leur masse :
- De 10% à 60% massique d'au moins une composition (C1) représentée par la formule (II) :

   R1-O-(G)ₓ-H (II)

   dans laquelle x représente un nombre décimal compris entre 1,05 et 2,5, G représente le reste d'un sucre réducteur, et R1 représente un radical aliphatique hydrocarboné, linéaire ou ramifié, saturé ou insaturé comportant de 12 à 36 atomes de carbone, ladite composition (C1) consistant en un mélange de composés représentés par les formules (I₁), (I₂), (I₃), (I₄) et (I₅) :

   R1-O-(G)₁-H (II₁)

   R1-O-(G)₂-H (II₂)

   R1-O-(G)₃-H (II₃)

   R1-O-(G)₄-H (II₄)

   R1-O-(G)₅-H (II₅)

   dans les proportions molaires respectives a₁, a₂, a₃, a₄ et a₅, telles que :
   - La somme a₁ + a₂ + a₃ + a₄ + a₅ est égale à 1 et
   - La somme a₁ + 2a₂ + 3a₃ + 4a₄ + 5a₅ est égale à x ;
   - De 40% à 90% massique d'au moins un alcool gras de formule (III) :

      R'1-OH (III),
dans laquelle R'1 représente un radical aliphatique hydrocarboné, linéaire ou ramifié, saturé ou insaturé comportant de 12 à 40 atomes de carbone.

Selon un aspect encore plus particulier, l'invention a pour objet une émulsion (E₁) telle que définie précédemment caractérisée en ce que ledit système émulsionnant (S) consiste en une composition (C₂) pour laquelle :
- Dans la composition (C₁) représentée par la formule (II), x représente un nombre décimal compris entre 1,05 et 2,5, G représente le reste d'un sucre réducteur choisi parmi le glucose, le xylose et l'arabinose, et R1 représente un radical aliphatique hydrocarboné choisi parmi les radicaux n-dodécyle, n-tétradécyle, n-hexadécyle, n-octadécyle, n-eicosyle, n-docosyle , n-oléyle, n-linoléyle ou n-linolényle ;
- Dans la formule (III), R'1 représente un radical aliphatique hydrocarboné choisi parmi les radicaux n-dodécyle, n-tétradécyle, n-hexadécyle, n-octadécyle, n-eicosyle, n-docosyle , n-oléyle, n-linoléyle ou n-linolényle.

Selon un aspect encore plus particulier, l'invention a pour objet une émulsion (E₁) telle que définie précédemment caractérisée en ce que :
- Dans la composition de formule (C1) représentée par la formule (II), x représente un nombre décimal compris entre 1,05 et 2,5, G représente le reste du glucose, et R1 représente un radical choisi parmi les radicaux dodécyle, tétradécyle, hexadécyle, octadécyle, eicosyle, béhényle, oléyle, linoléyle ou linolényle ; et
- Dans la formule (III), R'1 représente un radical aliphatique hydrocarboné choisi parmi les radicaux dodécyle, tétradécyle, hexadécyle, octadécyle, eicosyle, béhényle, oléyle, linoléyle ou linolényle.

Dans la définition de l'émulsion (E₁) objet de la présente invention, on désigne par acides gras polyéthoxylés comportant de 10 à 40 atomes de carbone et ayant un degré d'éthoxylation compris entre 5 et 100, un composé de formule (IV) : 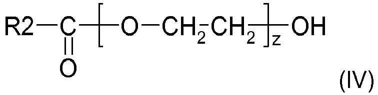 dans laquelle :
- R2-C(=O) représente un radical acyle saturé ou insaturé, linéaire ou ramifié comportant de 10 à 40 atomes de carbone, et plus particulièrement un radical choisi parmi les radicaux n-décanoyle, n-dodécanoyle, n-tétradécanoyle, n-hexadécanoyle, n-octadécanoyle, n-eicosanoyle, n-docosanoyle, n-oléyle, n-linoléyle, n-linolénoyle ou isostéaryle, et
- z représente un nombre entier supérieur ou égal à 5 et inférieur ou égal à 100, et plus particulièrement supérieur ou égal à 5 et inférieur ou égal à 80.

Selon un aspect plus particulier, le composé de formule (IV) est choisi parmi le stéarate de PEG-5, le stéarate de PEG-9, le stéarate de PEG-20, le stéarate de PEG-30, le stéarate de PEG-40, le stéarate de PEG-50, le stéarate de PEG-60, le stéarate de PEG-80, le stéarate de PEG-100, le palmitate de PEG-5, le palmitate de PEG-9, le palmitate de PEG-20, le palmitate de PEG-30, le palmitate de PEG-40, le palmitate de PEG-50, le palmitate de PEG-60, le palmitate de PEG-80, le palmitate de PEG-100, le laurate de PEG-5, le laurate de PEG-9, le laurate de PEG-20, le laurate de PEG-30, le laurate de PEG-40, le laurate de PEG-50, le laurate de PEG-60, le laurate de PEG-80, le laurate de PEG-100, l'oléate de PEG-5, l'oléate de PEG-9, l'oléate de PEG-20, l'oléate de PEG-30, l'oléate de PEG-40, l'oléate de PEG-50, l'oléate de PEG-60, l'oléate de PEG-80, l'oléate de PEG-100.

Dans la définition de l'émulsion (E₁) objet de la présente invention, on désigne par ester de glycérol d'acides gras comportant de 10 à 40 atomes de carbone, un composé de formule (V) : 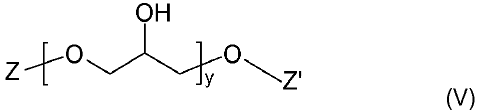 dans laquelle Z représente un radical acyle de formule R₃-C(=O)-, dans laquelle R₃ représente un radical aliphatique hydrocarboné, saturé ou insaturé, linéaire ou ramifié, comportant de 9 à 39 atomes de carbone et plus particulièrement un radical acyle choisi parmi les radicaux n-lauroyle, n-dodécanoyle, n-tétradécanoyle, n-hexadécanoyle, n-octadécanoyle, n-eicosanoyle, n-docosanoyle, n-oléyle, n-linoléyle, n-linolénoyle ou isostéaryle, Z' représente le radical acyle de formule R₃-C(=O)- tel que défini ci-dessus, avec Z' identique ou différent de Z, ou l'atome d'hydrogène, et y représente un nombre entier supérieur ou égal à 2 et inférieur ou égal à 20.

Selon un aspect plus particulier, le composé de formule (V) est choisi parmi l'oléate de décaglycérol, l'isostéarate de décaglycérol, le monolaurate de décaglycérol, le monolinoléate de décaglycérol, le mono-myristate de décaglycérol.

Dans la définition de l'émulsion (E₁) objet de la présente invention, on désigne par alcool gras polyéthoxylé comportant de 10 à 40 atomes de carbone et ayant un degré d'éthoxylation compris entre 2 et 100, un composé de formule (VI) : 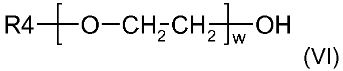 dans laquelle :
- R4 représente un radical aliphatique hydrocarboné, saturé ou insaturé, linéaire ou ramifié, comportant de 10 à 40 atomes de carbone, éventuellement substitué avec un ou plusieurs groupes hydroxyle. On désigne pour le radical R₄ dans la formule (VI) telle que définie ci-dessus, les radicaux alkyle linéaires saturés, par exemple les radicaux n-décyle, n-dodécyle, n-tridécyle, n-tétradécyle, n-pentadécyle, n-hexadécyle, n-heptadécyle, n-octadécyle, n-nonadécyle, n-eicosyle, n-docosyle ; les radicaux linéaires insaturés tels que les radicaux dodécènyle, tridécènyle, tétradécènyle, pentadécènyle, hexadécènyle, heptadécènyle, octadécènyle, nonadécènyle, eicosènyle, docosènyle, 4-dodécènyle, ou 5-dodécènyle ; les radicaux aliphatiques saturés ou insaturés, linéaires ou ramifiés, comportant de 10 à 40 atomes de carbone substitués par un ou deux groupes hydroxy, tels que les radicaux hydroxydodécyle, hydroxytétradécyle, hydroxyhexadécyle, hydroxyoctadécyle, Hydroxyeicosyle ou hydroxydocosyle, par exemple le radical 12-hydroxy octadécyle ; les radicaux issus des isoalcanols, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ; les radicaux alkyles ramifiés, issus des alcools de Guerbet, par exemple les radicaux 2-butyl octyle, 2-butyl décyle, 2-hexyl octyle, 2-hexyl décyle, 2-octyl décyle, 2-hexyl dodécyle, 2-octyl dodécyle, 2-décyl tétradécyle, 2-dodécyl hexadécyle ou 2-tétradécyl octadécyle,
- w représente un nombre entier supérieur ou égal à 2 et inférieur ou égal à 100, plus particulièrement supérieur ou égal à 2 et inférieur ou égal à 80, et encore plus particulièrement supérieur ou égal à 2 et inférieur ou égal à 50.

Dans la définition de l'émulsion (E₁) objet de la présente invention, on désigne par composition d'alcool gras polyéthoxylé comportant de 10 à 40 atomes de carbone et ayant un degré d'éthoxylation compris entre 2 et 100 et d'alcool gras comportant de 12 à 40 atomes de carbone, la composition (C₃) comprenant pour 100% de sa masse :
- De 20% à 80%, plus particulièrement de 30% à 70%, et encore plus particulièrement de 40% à 60% massique d'au moins un composé de formule (VI) telle que définie précédemment
- De 80% à 20%, plus particulièrement de 70% à 30% massique, et encore plus particulièrement de 60% à 40% massique d'au moins un alcool gras de formule (III) telle que définie précédemment.

Dans la définition de l'émulsion (E₁) objet de la présente invention, on désigne par composition d'acide gras polyéthoxylé, comportant de 10 à 40 atomes de carbone et ayant un degré d'éthoxylation compris entre 5 et 100, et d'ester de glycérol d'acide gras comportant de 10 à 40 atomes de carbone, et d'alcool gras comportant de 12 à 40 atomes de carbone, la composition (C₄) comprenant pour 100% de sa masse :
- De 10% à 40%, plus particulièrement de 15% à 35%, et encore plus particulièrement de 20% à 30% massique d'au moins un composé de formule (IV) telle que définie précédemment, et de 10% à 40%, plus particulièrement de 15% à 35%, et encore plus particulièrement de 20% à 30% massique d'au moins un composé de formule (V) telle que définie précédemment, et
- De 20% à 80%, plus particulièrement de 30% à 70%, et encore plus particulièrement de 40% à 60% massique d'au moins un composé de formule (III) telle que définie précédemment.

Selon un autre aspect particulier, l'invention a pour objet l'émulsion (E₁) telle que définie précédemment caractérisée en ce qu'elle comprend en outre, pour 100% de sa masse,
e) - Une proportion massique supérieure ou égale à 0,01% et inférieure ou égale à 0,5% d'au moins un polysaccharide (PS) choisi parmi les polysaccharides constitués de dérivés d'oses et par les polysaccharides constitués uniquement d'oses.

Les polysaccharides (PS) sont des polymères de saccharides. La définition IUPAC des saccharides désigne des oses, des composés d'oses proprement dits et leurs dérivés obtenus soit par réduction d'un groupement carbonyle, soit par oxydation d'une ou plusieurs fonctions hydroxyles, soit par le remplacement d'une ou plusieurs fonctions hydroxyles par un atome d'hydrogène, par un groupement amine, une fonction phosphate, une fonction sulfate.

Les polysaccharides les plus couramment utilisés pour la préparation de compositions alimentaires, cosmétiques ou pharmaceutiques, sont majoritairement constitués d'oses, tels que le glucose, le galactose, le mannose ou de dérivés d'oses pour lesquels la fonction hydroxyle du carbone terminal a été oxydée en fonction carboxyle. Par conséquent, on peut distinguer parmi les polysaccharides deux groupes distincts : les polysaccharides constitués uniquement d'oses (ou polyoses) et les polysaccharides constitués de dérivés d'oses.

Parmi les polysaccharides composés uniquement d'oses, on peut distinguer les glucanes, qui sont des homopolymères de glucose très abondants dans la nature, les glucomannoglycanes, les xyloglycanes et les galactomannanes, qui sont des polymères dont la chaîne principale est constituée d'unités de D-mannose, reliées entre elles en β-1,4, et sur laquelle des unités de D-galactose sont greffées latéralement par des liaisons α-1,6.

Les galactomannanes sont présents dans plusieurs espèces végétales, et plus particulièrement dans les espèces légumineuses dans lesquelles ils constituent l'albumen des graines. Selon leur origine végétale, le degré de substitution (DS) des unités de D-galactose sur la chaîne principale de D-mannose des galactomannanes, varie entre 0 et 1 :
- Les galactomannanes provenant de la gomme de cassia présentent un degré de substitution (DS) d'environ 1/5, signifiant le greffage latéral d'une unité de D-galactose toutes les 5 unités de D-mannose présentes sur la chaîne principale du polysaccharide ;
- Les galactomannanes provenant de la gomme de caroube présentent un degré de substitution (DS) d'environ 1/4, signifiant le greffage latéral d'une unité de D-galactose toutes les 4 unités de D-mannose présentes sur la chaîne principale du polysaccharide ;
- Les galactomannanes provenant de la gomme de tara présentent un degré de substitution (DS) d'environ 1/3, signifiant le greffage latéral d'une unité de D-galactose toutes les 3 unités de D-mannose présentes sur la chaîne principale du polysaccharide ;
- Les galactomannanes provenant de la gomme de guar présentent un degré de substitution (DS) d'environ 1/2, signifiant le greffage latéral d'une unité de D-galactose toutes les 2 unités de D-mannose présentes sur la chaîne principale du polysaccharide ;
- Les galactomannanes provenant de la gomme de fenugrec présentent un degré de substitution (DS) d'environ 1/1, signifiant le greffage latéral d'une unité de D-galactose pour quasiment toutes les unités de D-mannose présentes sur la chaîne principale du polysaccharide.

Parmi les polysaccharides constitués de dérivés d'oses, on peut distinguer :
- Les galactanes sulfatés, qui sont des polymères de galactose pouvant avoir des groupements esters-sulfate appendus, représentés notamment par les polyosides algaux comme les carraghénanes et l'agar ;
- Les uronanes, qui sont les polymères d'acides uroniques comme les algines et les pectines ;
- Les hétéropolymères d'oses et d'acides uroniques : souvent de composition complexe, ces polymères se trouvent notamment dans les exsudats de sève (comme par exemple l'exsudat de la gomme arabique et l'exsudat de la gomme de karaya) mais ils sont produits aussi par des microorganismes, comme par exemple la gomme xanthane et la gomme gellane ;
- Les glucosaminoglycanes qui sont des polyosides formés à partir d'un glucose dérivé par remplacement de son hydroxyle sur C-2 par une amine (appelé 2-amino-2-désoxy-D-glucose ou, plus simplement, glucosamine). La fonction amine peut être d'ailleurs acétylée.

Parmi les hydrocolloïdes dans cette classe on trouve le chitosane, formé uniquement de motifs glucosamine, et l'hyaluronane, dont l'unité de répétition est un dimère de glucosamine et d'acide glucuronique.

Selon un aspect plus particulier, l'invention a pour objet l'émulsion (E₁) telle que définie précédemment caractérisée en ce que le polysaccharide (PS) est choisi parmi les glucanes, les glucomannoglycanes, les xyloglycanes et les galactomannanes, les galactanes sulfatés, les uronanes, les hétéropolymères d'oses et d'acides uroniques, les glucosaminoglycanes.

Selon un aspect plus particulier, l'invention a pour objet l'émulsion (E₁) telle que définie précédemment, caractérisée en ce que le polysaccharide (PS) est choisi parmi le groupe constitué par les carraghénanes, l'agar, l'exsudat de la gomme arabique, l'exsudat de la gomme de karaya, la gomme xanthane, la gomme gellane, le chitosane, les glucoanes, les galactomannanes provenant de la gomme de cassia, les galactomannanes provenant de la gomme de caroube, les galactomannanes provenant de la gomme de tara, les galactomannanes provenant de la gomme de guar et les galactomannanes provenant de la gomme de fenugrec

Comme exemple particulier d'une telle émulsion (E₁) telle que définie ci-dessus, il y celle caractérisée en ce qu'elle comprend pour 100% de sa masse :
a) - De 52,5% à 76,4% massique d'une phase aqueuse (A₁) cosmétiquement acceptable ;
b) - De 0,5% à 2% massique d'un polyélectrolyte anionique réticulé (P) issu de la polymérisation, en présence d'au moins un agent de réticulation, de 60% à 80% molaire d'unités monomériques issues de l'acide 2-méthyl 2-[(1-oxo 2-propènyl) amino] 1-propanesulfonique partiellement ou totalement salifié sous forme de sel d'ammonium, avec de 15% molaire à 39,5% molaire d'unités monomériques issues du N,N-diméthyl acrylamide, et de 0,5% à 5% molaire d'unités monomériques issues du méthacrylate de lauryle tétraéthoxylé,
c) - De 8% à 15% massique d'au moins un agent épaississant inorganique (Ei) choisi parmi les parmi la kaolinite, la montmorillonite, la saponite, la bentonite ou l'hectorite.
d) - De 15% à 30% massique d'une phase grasse (A₂) comprenant pour 100% de sa masse :
   d₁) - De 5% à 25% massique d'un système émulsionnant (S) comprenant un ou plusieurs tensioactifs émulsionnants sélectionnés parmi les compositions d'alkylpolyglycosides ; les compositions d'alkylpolyglycosides et d'alcools gras ; les acides gras polyéthoxylés comportant de 10 à 40 atomes de carbone et ayant un degré d'éthoxylation compris entre 5 et 100 ; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone ; les alcools gras polyéthoxylés comportant de 10 à 40 atomes de carbone et ayant un degré d'éthoxylation compris entre 2 et 100 ; les compositions d'alcools gras polyéthoxylés comportant de 10 à 40 atomes de carbone et ayant un degré d'éthoxylation compris entre 2 et 100 et d'alcools gras comportant de 12 à 40 atomes de carbone; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone et d'alcools gras comportant de 12 à 40 atomes de carbone ; et
   d₂) - De 75% à 95% massique d'au moins une huile et optionnellement d'au moins une cire ;
e) - De 0,1% à 0,5% massique d'au moins un polysaccharides choisi parmi l'agar, l'exsudat de la gomme arabique, l'exsudat de la gomme de karaya, la gomme xanthane, la gomme gellane, les galactomannanes provenant de la gomme de cassia, les galactomannanes provenant de la gomme de caroube, les galactomannanes provenant de la gomme de tara, les galactomannanes provenant de la gomme de guar et les galactomannanes provenant de la gomme de fenugrec,
   ladite émulsion (E₁) étant en outre caractérisée en ce que le rapport massique entre l'agent épaississant inorganique (Ei) et le polyélectrolyte anionique réticulé (P) est supérieur ou égal à 8/1 et inférieur ou égal à 20/1.

L'émulsion (E₁) objet de la présente invention telle que définie précédemment est destinée à un usage topique, et peut être incorporée dans tout type de formulation cosmétique, dermocosmétique, dermopharmaceutique ou pharmaceutique destinée à un usage topique, ou bien encore dans tout type de support destiné à être mis en contact avec la peau (papier, lingette, textile, dispositif transdermique, etc.).

L'expression "à usage topique" signifie que l'émulsion (E₁) est mise en oeuvre par application sur la peau, les cheveux, le cuir chevelu ou les muqueuses, qu'il s'agisse d'une application directe dans le cas d'une formulation cosmétique, dermocosmétique, dermopharmaceutique ou pharmaceutique ou d'une application indirecte par exemple dans le cas d'un produit d'hygiène corporelle, de soin ou de protection de la peau, se présentant sous la forme d'un article en textile, par exemple une lingette, ou en papier, par exemple un papier à usage sanitaire.

L'émulsion (E₁) objet de la présente invention peut être conditionnée dans un flacon à ouverture totale ou partielle, dans un dispositif de type « flacon pompe », dans un dispositif muni d'une paroi ajourée par exemple une grille, dans un dispositif muni d'un applicateur a billes (dit"roll-on).

L'émulsion (E₁) objet de la présente invention peut être utilisée comme laits nettoyants ou démaquillants, comme lotions nettoyantes ou démaquillantes, comme agent nettoyant pour le visage ou pour le corps, comme shampooing pour le nettoyage des cheveux et/ou du cuir chevelu, comme après-shampooing pour le traitement des cheveux et/ou du cuir chevelu, comme mousse pour le soin ou pour la protection du visage, des mains et du corps, par exemple comme agent protecteur des rayonnements solaires, comme agent auto-bronzant, comme agent anti-âge, comme agent antirides, comme agent apaisant, comme agent hydratant.

L'émulsion (E₁) objet de la présente invention peut en outre comporter des excipients et/ou des principes actifs habituellement mis en oeuvre dans le domaine des formulations à usage topique, en particulier cosmétiques, dermocosmétiques, pharmaceutiques ou dermopharmaceutique.

L'émulsion (E₁) objet de la présente invention et telle que définie précédemment, peut comprendre en outre, un ou plusieurs composés auxiliaires chois parmi les tensioactifs moussants et/ou détergents, les tensioactifs épaississants et/ou gélifiants, les agents stabilisants, les composés filmogènes, les solvants et cosolvants, les agents hydrotropes, les agents plastifiants, les agents opacificants, les agents nacrants, les agents surgraissants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents, éventuellement présents dans l'émulsion (E₁) objet de la présente invention, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à l'émulsion (E₁) objet de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents éventuellement présents dans l'émulsion (E₁) objet de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans l'émulsion (E₁) objet de la présente invention, on peut citer particulièrement les dérivés d'ammoniums quaternaires.

Parmi les tensioactifs non ioniques moussants et/ou détergents éventuellement présents dans l'émulsion (E₁) objet de la présente invention, on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines.

Comme exemples de tensioactifs épaississants et/ou gélifiants éventuellement présents dans l'émulsion (E₁) objet de la présente invention, on peut citer :
- Les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ;
- Les esters gras alcoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141 ;
- Les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples de tensioactifs émulsionnants éventuellement présents dans l'émulsion (E₁) objet de la présente invention, on peut citer des tensioactifs non ioniques, des tensioactifs anioniques, des tensioactifs cationiques.

Comme exemples de tensioactifs non ioniques émulsionnants éventuellement présents dans l'émulsion (E₁) objet de la présente invention, on peut citer les esters d'acides gras et de sorbitol, par exemple les produits commercialisés sous les appellations MONTANE™80 et MONTANE™85 et MONTANE™60; l'huile de ricin éthoxylée et l'huile de ricin hydrogénée éthoxylée, par exemple le produit commercialisé sous la dénomination SIMULSOL™ 989; les compositions comprenant du stéarate de glycérol et de d'acide stéarique poly(éthoxylé) avec entre 5 moles et 150 moles d'oxyde d'éthylène, par exemple la composition comprenant de l'acide stéarique (éthoxylé) à 135 moles d'oxyde d'éthylène et du stéarate de glycérol commercialisée sous l'appellation SIMULSOL™ 165 ; les esters de sorbitan éthoxylés, par exemple les produits commercialisés sous la dénomination MONTANOX™ ; les esters de mannitan ; les esters de mannitan éthoxylés ; les esters de sucrose ; les esters de méthylglucoside.

Comme exemples de tensioactifs anioniques émulsionnants éventuellement présents dans l'émulsion (E₁) objet de la présente invention, on peut citer le décylphosphate, le cétylphosphate commercialisé sous l'appellation AMPHISOL™, le glycéryl stéarate citrate ; le cétéarylsulfate ; la composition arachidyl/béhényl phosphates et arachidyl/béhényl alcools commercialisée sous l'appellation SENSANOV™WR; les savons par exemple le stéarate de sodium ou le stéarate de triéthanolammonium, les dérivés N-acylés d'acides aminés salifiés comme par exemple le stéaroyl glutamate.

Comme exemples de tensioactifs cationiques émulsionnants éventuellement présents dans l'émulsion (E₁) objet de la présente invention, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants éventuellement présents dans l'émulsion (E₁) objet de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture éventuellement présents dans l'émulsion (E₁) objet de la présente invention, on peut citer des dérivés N-acylés d'acides aminés, par exemple la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica.

Comme exemples de solvants et de co-solvants éventuellement présents dans l'émulsion (E₁) objet de la présente invention, on peut citer l'eau, les solvants organiques par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales éventuellement présentes dans l'émulsion (E₁) objet de la présente invention, on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes éventuellement présents dans l'émulsion (E₁) objet de la présente invention, on peut citer les xylènes sulfonates, les cumènes sulfonates, l'hexyl polyglucoside, le (2-éthyl hexyl) polyglucoside ou le n-heptyl polyglucoside.

Comme exemples d'agents déodorants éventuellement présents dans l'émulsion (E₁) objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de principes actifs éventuellement présents dans l'émulsion (E₁) objet de la présente invention, on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecylènoyl phénylalanine commercialisé sous l'appellation SEPIWHITE™MSH, le SEPICALM™VG, le mono ester et/ou le diester de glycérol du ω-undecelynoyl phénylalanine, les ω-undecylènoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylglucoside, la composition commercialisée sous le nom de marque AQUAXYL™, la composition commercialisés sous le nom de marque PRO-XYLANE™, les dérivés de C-glycosides et plus particulièrement les dérivés de C-glucosides, de C-xylosides ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5, le FLUIDIPURE™8G ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de centalla asiatica, de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caraténoïdes (et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultraviolets, par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI: Acetyl Tyrosine, Monk's pepper extract (Vitex Agnus-castus) par la société Mibelle qui contient de l'acétyl tyrosine, produit commercialisé sous le nom de marque Unipertan VEG-24/242/2002 (nom INCI: butylene glycol and Acetyl Tyrosine and hydrolyzed vegetable protein and Adenosine triphosphate) par la société UNIPEX, le produit commercialisé sous le nom de marque « Try-Excell™ » (nom INCI: Oleoyl Tyrosine and Luffa Cylindrica (Seed Oil and Oleic acid) par la société Sederma qui contient des extraits de pépins de courge (ou huile de Loofah), le produit commercialisé sous le nom de marque «Actibronze™» (nom INCI: hydrolyzed wheat protein and acetyl tyrosine and copper gluconate) par la société Alban Muller, le produit commercialisé sous le nom de marque Tyrostan™ (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze™ (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI: Butylene glycol , Palmitoyl Tripeptide-40) par la société LIPOTEC, les sucres et les dérivés de sucres par exemple le produit commercialisé sous le nom de marque Tanositol™ (nom INCI: inositol) par la société Provital, le produit commercialisé sous le nom de marque Thalitan™ (ou Phycosaccharide™ AG) par la société CODIF international (nom INCI: Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI: Maltodextrin, Mucuna Pruriens Seed extract) par la société Alban Muller, les composés riches en flavonoïdes par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP 1 515 688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C, et plus généralement ceux cités dans la demande de brevet européen publiée sous le numéro EP 1 515 688 A2.

Comme exemples d'agents antioxydants éventuellement présents dans l'émulsion (E₁) objet de la présente invention, on peut citer l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE™ GL 47S commercialisé par la société Akzo Nobel sous le nom INCI: Tetrasodium Glutamate Diacetate.

Comme exemples de filtres solaires éventuellement présents dans l'émulsion (E₁) objet de la présente invention, on peut citer tous ceux figurant dans la directive cosmétique 76/768/CEE modifiée annexe VII.

Parmi les filtres organiques solaires éventuellement présents dans l'émulsion (E₁) objet de la présente invention, on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de (p-isopropanol phényle) ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle, le cinnamate de (p-méthoxy 2-éthylhexyle), le cinnamate de (p-méthoxy 2-éthoxy éthyle), le cinnamate de (p-méthoxy cyclohexyle), le cinnamate d'éthyl-α-cyano-β-phényle, le cinnamate de (2-éthyl hexyl)-α-cyano-β-phényle, le cinnamate de diparaméthoxy mono-2-éthylhexanoyl de glycéryle ; la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le (2-éthyl hexyl) 4'-phényl benzophénone-2-carboxylate, la 2-hydroxy 4-(n-octyloxy) benzophénone, la 4-hydroxy 3-carboxy benzophénone ; le 3-(4'-méthyl benzylidène) d,l-camphre, le 3-(benzylidène) d,l-camphre, le benzalkonium méthosulfate camphre ; l'acide urocanique, l'urocanate d'éthyle ; la famille des dérivés de l'acide sulfonique comme l'acide sulfonique 2-phényl benzimidazole-5 et ses sels ; la famille des dérivés de la triazine comme l'hydroxyphényl triazine, l'(éthylhexyloxy) (hydroxyphényl) (4-méthoxy phényl) triazine, le 2,4,6-trianillino-(p-carbo-2'-éthylhexyl-1'-oxy)-1,3,5-triazine, le 4,4-((6-(((1,1-diméthyl éthyl) amino) carbonyl) phenyl) amino)-1,3,5-triazine-2,4-diyl diimino) bis(2-éthyl hexyl) ester de l'acide benzoïque, le 2-phényl-5-méthyl benzoxazole, le 2-(2'-hydroxy 5'-méthyl phényl) benzotriazole, le 2-(2'-hydroxy 5'-t-octyl phényl) benzotriazole, le 2-(2'-hydroxy 5'-méthy phényl) benzotriazole; la dibenzazine; le dianisoylméthane, le 4-méthoxy 4"-t-butyl benzoyl méthane ; la 5-(3,3-diméthyl 2-norbornylidène)-3-pentan-2-one ; la famille des dérivés du diphénylacrylate comme le (2-éthyl hexyl) 2-cyano 3,3-diphényl 2-propènoate, l'éthyl-2-cyano 3,3-diphényl 2-propènoate ; la famille des polysiloxanes comme le malonate de benzylidène siloxane.

Parmi les filtres inorganiques solaires, également appelés "écrans minéraux", éventuellement présents dans l'émulsion (E₁) objet de la présente invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'invention a également pour objet un procédé de préparation d'une émulsion (E₁) de type huile-dans-eau se présentant sous la forme d'une mousse et telle que définie précédemment, comprenant :
Au moins une étape a) de préparation d'une phase (A') par mélange du polyélectrolyte anionique réticulé (P), et optionnellement du polysaccharide (PS), dans la phase grasse (A₂) ; et
Au moins une étape b) d'émulsification de ladite phase (A') obtenue à l'issue de l'étape a) avec la phase aqueuse (A₁) cosmétiquement acceptable pour obtenir une émulsion (E'₁) ;et
Au moins une étape c) d'obtention de l'émulsion (E₁) par mélange de l'agent épaississant inorganique (Ei) dans l'émulsion (E'₁) obtenue à l'issue de l'étape b)
L'étape a) de préparation d'une phase (A') par mélange du polyélectrolyte anionique réticulé (P), et optionnellement du polysaccharide (PS), dans la phase grasse (A₂), est généralement conduite à une température supérieure ou égale à 20°C et inférieure ou égale à 85°C, plus particulièrement supérieure ou égale à 20°C et inférieure ou égale à 80°C, et encore plus particulièrement supérieure ou égale à 40°C et inférieure ou égale à 80°C ; elle est réalisée sous agitation mécanique à une vitesse modérée supérieure ou égale à 50 tours/minute et inférieure ou égale à 100 tours/minute.
L'étape b) d'émulsification de ladite phase (A'), obtenue à l'issue de l'étape a) avec la phase aqueuse (A₁) cosmétiquement acceptable pour obtenir une émulsion (E'₁), est généralement conduite à une température supérieure ou égale à 20°C et inférieure ou égale à 85°C, plus particulièrement supérieure ou égale à 20°C et inférieure ou égale à 80°C, et encore plus particulièrement supérieure ou égale à 40°C et inférieure ou égale à 80°C ; elle est réalisée sous agitation mécanique par l'intermédiaire d'un dispositif mécanique cisaillant, comme par exemple un système rotor-stator, à une vitesse de cisaillement supérieure ou égale à 1 000 tours/minute et inférieure ou égale à 8 000 tours/minutes, plus particulièrement supérieure ou égale à 2 000 tours/minute et inférieure ou égale à 8 000 tours/minutes, et encore plus particulièrement supérieure ou égale à 4 000 tours/minute et inférieure ou égale à 8 000 tours/minutes.
L'émulsion (E'₁) obtenue à l'issue de la mise en oeuvre de l'étape b) du procédé objet de la présente invention présente un aspect d'émulsion huile-dans-eau lisse et ne présente pas de bulles de gaz dispersées : l'émulsion (E'₁) obtenue à l'issue de la mise en oeuvre de ladite étape b) ne se présente pas sous la forme d'une émulsion huile-dans-eau sous forme de mousse.
L'émulsion (E'₁) obtenue à l'issue de la mise en oeuvre de l'étape b) du procédé objet de la présente invention peut être optionnellement être mise sous atmosphère d'un gaz autre que l'air, par inertage du ciel du réacteur contenant l'émulsion (E'₁) ; ledit inertage consistant en un balayage de l'atmosphère par le gaz choisi parmi l'azote, le dioxyde de carbone, l'oxygène ou l'argon et aboutissant à la substitution de l'atmosphère d'air par une atmosphère dudit gaz.
L'étape c) d'obtention de l'émulsion (E₁) par mélange de l'agent épaississant inorganique (Ei) dans l'émulsion (E'₁) obtenue à l'issue de l'étape b) est généralement conduite à une température supérieure ou égale à 20°C et inférieure ou égale à 85°C, plus particulièrement supérieure ou égale à 20°C et inférieure ou égale à 80°C, et encore plus particulièrement supérieure ou égale à 40°C et inférieure ou égale à 80°C ; elle est réalisée sous agitation mécanique par l'intermédiaire d'un dispositif mécanique cisaillant, comme par exemple un système rotor-stator, à une vitesse de cisaillement supérieure ou égale à 1 000 tours/minute et inférieure ou égale à 8 000 tours/minutes, plus particulièrement supérieure ou égale à 2 000 tours/minute et inférieure ou égale à 8 000 tours/minutes, et encore plus particulièrement supérieure ou égale à 4 000 tours/minute et inférieure ou égale à 8 000 tours/minutes.

L'émulsion (E₁) obtenue à l'issue de la mise en oeuvre de l'étape c) du procédé objet de la présente invention est une émulsion huile-dans-eau qui se présente sous la forme d'une mousse.

L'émulsion (E₁) obtenue à l'issue de la mise en oeuvre de l'étape c) du procédé objet de la présente invention peut être ensuite refroidie pour atteindre une température inférieure ou égale à 35°C.

L'invention a pour aussi pour objet l'utilisation de l'émulsion (E₁) telle que définie précédemment, pour le traitement cosmétique de la peau, des cheveux, du cuir chevelu, des muqueuses et/ou les ongles.

Selon un aspect particulier l'invention a pour objet l'utilisation de l'émulsion (E₁) telle que décrite précédemment, pour le nettoyage, et/ou pour le soin de la peau, des cheveux, du cuir chevelu, des muqueuses et/ou des ongles.

Par utilisation de l'émulsion (E₁) pour le soin de la peau, on entend dans le cadre de la présente invention toute utilisation visant à hydrater les couches supérieures de la peau humaine (épiderme et derme), visant à protéger la peau humaine contre les rayonnements UV-A et UV-B de sources lumineuse comme le soleil, visant à colorer artificiellement la peau humaine pour la faire brunir, visant à éclaircir la peau humaine, visant à diminuer ou à éliminer les effets inesthétiques liés à la présence de cernes ou de poches péri-oculaires, visant à diminuer ou à éliminer toute inflammation de la peau humaine, visant à prévenir ou diminuer ou éliminer toute contamination bactérienne ou fongique de la peau humaine, et visant à empêcher ou à ralentir l'apparition des signes du vieillissement de la peau humaine ou bien d'éliminer lesdits signes ; lesdits signes de vieillissement de la peau humaine ou des lèvres sont le manque d'élasticité et/ou de tonus de la peau humaine ou des lèvres, ou le manque de densité et/ou de fermeté de la peau humaine ou des lèvres, ou une altération du microrelief de la peau humaine ou des lèvres.

Selon un autre aspect particulier, l'invention a pour objet l'utilisation de l'émulsion (E₁) telle que décrite précédemment, pour le maquillage de la peau, des cheveux et/ou des ongles.

Les exemples suivants illustrent l'invention sans toutefois la limiter.

### I) - Préparation d'un polyélectrolyte anionique réticulé (P)

### Terpolymère de 2-méthyl 2-[(1-oxo 2-propènyl) amino] 1-propanesulfonate d'ammonium, de N,N-diméthyl acrylamide et de méthacrylate de lauryle tétraéthoxylé [AMPSNH₄/DMAM/MAL(4OE) 77,4/19,2/3,4 molaire], réticulé au propanetriacrylate de triméthylol (TMPTA).

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium (AMPSNH₄) dans un mélange tert-butanol/eau (97,5/2,5 en volume), 10,1g de N,N-diméthyl acrylamide (DMAM), 4,2g de méthacrylate de lauryle tétra-éthoxylé [MAL(4OE)] et 0,75g de TMPTA. Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : "Polyélectrolyte (P)".

### II) - Préparation et évaluation d'émulsions huile-dans-eau se présentant sous la forme d'une mousse selon l'invention et d'émulsions huile-dans-eau comparatives.

### II-1 Préparation des émulsions huile-dans-eau se présentant sous forme de mousse (selon l'invention) et d'émulsions huile-dans-eau comparatives

On prépare 10 émulsions huile-dans-eau selon l'invention, notées (F1) à (F10), dont les proportions massiques de leurs constituants sont indiquées dans le tableau 1 DES émulsions huile-dans-eau comparatives notées (F'1) à (F'7) dont les proportions massiques de leurs constituants sont indiquées dans le tableau 2 ci-dessous. Le procédé de préparation commun pour les émulsions huile-dans-eau (F1) à (F10) et pour les émulsions huile-dans-eau (F'1) à (F'4), est le suivant :
- On verse dans un bécher, à une température de 85°C, les différents constituants de la phase grasse sous agitation mécanique à une vitesse de quatre-vingt tours par minute,
- On disperse progressivement et successivement dans le bêcher contenant la phase grasse le Polyélectrolyte (P) (ou selon les cas le Sepiplus™ 400 ou le Pemulen™ TR2), et le Solagum™AX sous agitation mécanique à une vitesse de quatre-vingt tours par minute ;
- On verse dans un autre bécher, à une température de 85°C, les différents constituants de la phase aqueuse sous agitation mécanique à une vitesse de quatre-vingt tours par minute ;
- Le contenu du bêcher comprenant la phase aqueuse est progressivement ajouté à la phase grasse, comprenant le système polymérique, à une température de 85°C, sous agitation mécanique au moyen d'un mobile d'agitation muni d'un système rotor-stator à quatre mille tours par minute pendant quatre minutes ;
- L'épaississant organique (kaolin ou argile rouge ou argile verte), préalablement mis sous la forme d'une dispersion aqueuse, est ensuite introduit sur le mélange précédemment formé à une température de 85°C ;
- Le mélange ainsi obtenu est ensuite soumis à une agitation mécanique cisaillante au moyen d'un mobile d'agitation muni d'un système rotor-stator à quatre mille tours par minute pendant quatre minutes ; puis refroidi à une température de 25°C, puis vidangé pour obtenir les émulsions à évaluer.

Le procédé de préparation de l'émulsion huile-dans-eau comparative (F'5) est le suivant :
- On verse dans un bécher, à une température de 85°C, les différents constituants de la phase grasse sous agitation mécanique à une vitesse de quatre-vingt tours par minute,
- On disperse progressivement et successivement dans le bêcher contenant la phase grasse le Polyélectrolyte (P) et le Solagum™AX sous agitation mécanique à une vitesse de quatre-vingt tours par minute ;
- On verse dans un autre bécher, à une température de 85°C, les différents constituants de la phase aqueuse sous agitation mécanique à une vitesse de quatre-vingt tours par minute ;
- Le contenu du bêcher comprenant la phase aqueuse est progressivement ajouté à la phase grasse, comprenant le système polymérique, à une température de 85°C, sous agitation mécanique au moyen d'un mobile d'agitation muni d'un système rotor-stator à quatre mille tours par minute ;
- Le mélange ainsi obtenu est refroidi à 20°C puis vidangé pour obtenir l'émulsion (F'5) à évaluer.

Le procédé de préparation de l'émulsion huile-dans-eau comparative (F'6) comprend l'ensemble des étapes mises en oeuvre dans le procédé de préparation de l'émulsion huile-dans-eau comparative (F'5), à l'exception de l'étape de vidange, suivies par une étape supplémentaire d'intoduction d'azote, à une température de 20°C, pendant une durée de trente minutes, au moyen d'un dispositif d'arrivée de gaz adapté. Le mélange ainsi obtenu est refroidi à 20°C puis vidangé pour obtenir l'émulsion (F'6) à évaluer.

Le procédé de préparation de l'émulsion huile-dans-eau comparative (F'7) comprend l'ensemble des étapes mises en oeuvre dans le procédé de préparation de l'émulsion huile-dans-eau comparative (F'5), à l'exception de l'étape de vidange, suivies par une étape supplémentaire d'intoduction d'azote, à une température de 60°C, pendant une durée de 30 minutes, au moyen d'un dispositif d'arrivée de gaz adapté. Le mélange ainsi obtenu est refroidi, toujours sous agitation et sous barbotage d'azote, pour atteindre une température de 20°C, puis l'introduction d'azote et l'agitation sont arrêtés pour vidanger et pour obtenir l'émulsion (F'7) à évaluer.

**Tableau 1**

| Emulsion | (F1) | (F2) | (F3) | (F4) | (F5) | (F6) |
|---|---|---|---|---|---|---|
| **Phase grasse** | | | | | | |
| Glycéryl stéarate | 2% | 2% | 2% | 2% | 2% | 2% |
| Néopentyl glycol diéthylhexanoate | 6% | 6% | 6% | 6% | 6% | 6% |
| Isononate d'isononyle | 6% | 6% | 6% | 6% | 6% | 6% |

| **Système émulsionnant** | | | | | | |
|---|---|---|---|---|---|---|
| Montanov 202 ⁽¹⁾ | 2% | 2% | 2% | 2% | 2% | 2% |
| Montanov 68 ⁽²⁾ | 1% | 1% | 1% | 1% | 1% | 1% |

| **Système polymérique** | | | | | | |
|---|---|---|---|---|---|---|
| Polyélectrolyte(P) | 0,5% | 1% | 1,5% | 1% | 1% | 1% |
| Solagum AX ⁽³⁾ | 0,2% | 0,2% | 0,2% | 0,2% | 0,2% | 0,2% |

| **Epaississant inorganique (Ei)** | | | | | | |
|---|---|---|---|---|---|---|
| Kaolin | 10% | 10% | 10% | 8% | 12% | 15% |
| Argile verte | 0% | 0% | 0% | 0% | 0% | 0% |
| Argile rouge | 0% | 0% | 0% | 0% | 0% | 0% |

| **Phase aqueuse** | | | | | | |
|---|---|---|---|---|---|---|
| Eau | Qs 100% | Qs 100% | Qs 100% | Qs 100% | Qs 100% | Qs 100% |
| Euxyl PE 9010⁽⁴⁾ | 1% | 1% | 1% | 1% | 1% | 1% |

| Emulsion | (F7) | (F8) | (F9) | (F10) | | |
|---|---|---|---|---|---|---|
| **Phase grasse** | | | | | | |
| Glycéryl stéarate | 2% | 2% | 2% | 2% | | |
| Néopentyl glycol diéthylhexanoate | 6% | 6% | 6% | 6% | | |
| Isononate d'isononyle | 6% | 6% | 6% | 6% | | |

| **Système émulsionnant** | | | | | | |
|---|---|---|---|---|---|---|
| Montanov 202 ⁽¹⁾ | 2% | 2% | 0% | 0% | | |
| Montanov 68 ⁽²⁾ | 1% | 1% | 0% | 0% | | |
| Brij™S2 ⁽⁹⁾ | 0% | 0% | 1,5% | 0% | | |
| Brij™S721 ⁽¹⁰⁾ | 0% | 0% | 1,5% | 0% | | |
| Alcools C16/C18 (50/50)**⁽⁵⁾** | 0% | 0% | 1% | 1% | | |
| Simulsol™ 165⁽⁶⁾ | 0% | 0% | 0% | 3% | | |

| **Système polymérique** | | | | | | |
|---|---|---|---|---|---|---|
| Polyélectrolyte (P) | 1% | 1% | 1% | 1% | | |
| Solagum AX ⁽³⁾ | 0,2% | 0,2% | 0,2% | 0,2% | | |

| **Epaississant inorganique (Ei)** | | | | | | |
|---|---|---|---|---|---|---|
| Kaolin | 0% | 0% | 10% | 10% | | |
| Argile verte | 10% | 0% | 0% | 0% | | |
| Argile rouge | 0% | 10% | 0% | 0% | | |

| **Phase aqueuse** | | | | | | |
|---|---|---|---|---|---|---|
| Eau | Qs 100% | Qs 100% | Qs 100% | Qs 100% | | |
| Euxyl PE 9010⁽⁴⁾ | 1% | 1% | 1% | 1% | | |

**Tableau 2**

| Emulsion | (F'1) | (F'2) | (F'3) | (F'4) | (F'5) | (F'6) | (F'7) |
|---|---|---|---|---|---|---|---|
| **Phase grasse** | | | | | | | |
| Glycéryl stéarate | 2% | 2% | 2% | 2% | 2% | 2% | 2% |
| Néopentyl glycol diéthylhexanoate | 6% | 6% | 6% | 6% | 6% | 6% | 6% |
| Isononate d'isononyle | 6% | 6% | 6% | 6% | 6% | 6% | 6% |

| **Système émulsionnant** | | | | | | | |
|---|---|---|---|---|---|---|---|
| Montanov 202 ⁽¹⁾ | 2% | 2% | 2% | 2% | 2% | 2% | 2% |
| Montanov 68 ⁽²⁾ | 1% | 1% | 1% | 1% | 1% | 1% | 1% |

| **Système polymérique** | | | | | | | |
|---|---|---|---|---|---|---|---|
| Polyélectrolyte_ (P) | 1% | 0% | 0% | 1% | 1% | 1% | 1% |
| Solagum AX ⁽³⁾ | 0,2% | 0,2% | 0,2% | 0,2% | 0,2% | 0,2% | 0,2% |
| Sepiplus 400⁽⁷⁾ | 0% | 1,7% | 0% | 0% | 0% | 0% | 0% |
| Pemulen TR2⁽⁸⁾ | 0% | 0% | 1% | 0,5% | 0% | 0% | 0% |

| **Epaississant inorganique (Ei)** | | | | | | | |
|---|---|---|---|---|---|---|---|
| Kaolin | 5% | 10% | 10% | 10% | 0% | 0% | 0% |

| **Phase aqueuse** | | | | | | | |
|---|---|---|---|---|---|---|---|
| Eau | Qs 100% | Qs 100% | Qs 100% | Qs 100% | Qs 100% | Qs 100% | Qs 100% |
| Euxyl PE 9010⁽⁴⁾ | 1% | 1% | 1% | 1% | 1% | 1% | 1% |

| | | | | | | | |
|---|---|---|---|---|---|---|---|
| (1) Montanov™ 202 (nom INCl : Arachidyl Alcohol & Behenyl Alcohol & Arachidyl Glucoside) : Agent émulsionnant commercialisé par la société SEPPIC ; (2) Montanov™ 68 (nom INCl : Cetearyl Alcohol & Cetearyl Glucoside) : Agent émulsionnant commercialisé par la société SEPPIC ; (3) Solagum™AX (nom INCI Acacia Senegal Gum & Xanthan Gum) : Agent épaississant commercialisé par la société SEPPIC ; (4) Euxyl™ PE 9010 (nom INCl : phenoxyethanol & ethylhexylglycerin) : Agent conservateur commercialisé par la société Schülke ; (5) Alcools C16/C18 (50/50) : Mélange d'hexadécanol-1 et d'octyldécanol-1, dans un ratio massique de 50/50 ; (6) Simulsol™ 165 (nom INCI : PEG-100 stearate & Glyceryl stearate) : Agent émulsionnant commercialisé par la société SEPPIC, comprenant pour 100% de sa masse une proportion massique de 50% de PEG-100 stéarate et une proportion massique de 50% de Glycéryl stéarate ; (7) Sepiplus™ 400 (nom INCl: Polyacrylate 13 & Polyisobutene & Polysorbate 20) : Epaississant polymérique se présentant sous la forme d'un latex inverse, comprenant un polymère réticulé comprenant de l'acrylamide, du 2-Acrylamido-2-méthylpropane sulfonate de sodium, de l'acide acrylique et de l'acrylate de sodium, dont la teneur massique en ledit polymère réticulé dans la latex inverse est égale à 60% ; (8) Pemulen™TR2 (Nom INCl : Acrylates/C10-30 Alkyl Acrylate Crosspolymer) : Emulsionnant polymérique de haut poids moléculaire, copolymère réticulé d'acide acrylique et d'un comonomère hydrophobe ; (9) Brij™S2 (nom INCl : steareth-2) : Agent émulsionnant provenant de l'éthoxylation de l'alcool stéarylique avec 2 moles d'oxyde d'éthylène, commercialisé par la société CRODA ; (10) Brij™S721 (nom INCl : steareth-21) : Agent émulsionnant provenant de l'éthoxylation de l'alcool stéarylique avec 21 moles d'oxyde d'éthylène, commercialisé par la société CRODA. | | | | | | | |

### II-2 Mise en évidence des propriétés des émulsions huile -dans- eau (F1) à (F10) selon l'invention et des émulsions comparatives huile -dans- eau (F'1) à (F'7).

### 112.1 Caractérisation de l'aspect et de la viscosité des émulsions huile -dans- eau (F1) à (F10) selon l'invention et des émulsions comparatives huile -dans- eau (F'1) à (F'7).

Les émulsions (F1) à (F10) selon l'invention et les émulsions comparatives huile -dans- eau (F'1) à (F'7) obtenues selon les procédés précédemment décrits, sont ensuite conservées dans une enceinte climatique isolée et régulée à une température de 20°C pendant un mois. A l'issue d'une durée d'un jour (J1), de 7 jours (J7) et d'un mois (M1), l'aspect **(ASP)** de chaque émulsion préparée est observé.

A l'issue d'une durée de 7 jours (J7) et d'un mois (M1), la viscosité dynamique **(µ)** de chaque émulsion est mesurée (en mPas) au moyen d'un viscosimètre à 20°C (Brookfield RVT, mobile 7 ; cinq tours par minute).

### Expression des résultats :

- lorsque l'émulsion observée se présente sous la forme d'une émulsion homogène sous forme de mousse, l'aspect (ASP) est noté « HMo » ;
- lorsque l'émulsion observée se présente sous la forme d'une émulsion homogène mais ne se trouve pas sous forme de mousse, l'aspect (ASP) est noté « HNMo » ;-lorsque l'émulsion observée se présente sous la forme d'une émulsion hétérogène du fait de la présence de grumeaux et ne se trouve pas sous la forme d'une moussse, l'aspect (ASP) est noté « Het-NMo ».

### 11-2.2 Résultats obtenus pour les émulsions huile -dans- eau (F1) à (F10) selon l'invention et des émulsions comparatives huile -dans- eau (F'1) à (F'7).

Les méthodes d'évaluations décrites au paragraphe II-2.1 ont été appliquées aux émulsions huile -dans-eau (F1) à (F10) selon l'invention et des émulsions comparatives huile -dans-eau (F'1) à (F'7). Les résultats obtenus sont consignés dans les tableaux 3 et 4 ci-dessous, respectivement pour les émulsions huile -dans- eau (F1) à (F10) selon l'invention et des émulsions comparatives huile -dans- eau (F'1) à (F'7).

**Tableau 3**

| | (F1) | (F2) | (F3) | (F4) | (F5) | (F6) |
|---|---|---|---|---|---|---|
| **ASP** (J1, 20°C) | HMo | HMo | HMo | HMo | HMo | HMo |
| **ASP** (J7, 20°C) | HMo | HMo | HMo | HMo | HMo | HMo |
| **µ** (J7) | 41 100 | 124 000 | 264 000 | 88 000 | 149 000 | 210 000 |
| **ASP** (M1) | HMo | HMo | HMo | HMo | HMo | HMo |
| **µ** (M1) | 41 200 | 118 000 | 238 000 | 108 000 | 179 000 | 204 000 |
| **ASP** (J7, 45°C) | HMo | HMo | HMo | HMo | HMo | HMo |
| **ASP** (M1, 45°C) | HMo | HMo | HMo | HMo | HMo | HMo |

| | (F7) | (F8) | (F9) | (F10) | | |
|---|---|---|---|---|---|---|
| **ASP** (J1, 20°C) | HMo | HMo | HMo | HMo | | |
| **ASP** (J7, 20°C) | HMo | HMo | HMo | HMo | | |
| **µ** (J7) | 160 000 | 240 000 | 135 000 | 161 000 | | |
| **ASP** (M1, 20°C | HMo | HMo | HMo | HMo | | |
| **µ** (M1) | 171 000 | 156 000 | 122 000 | 160 000 | | |
| **ASP** (J7, 45°C) | HMo | HMo | HMo | HMo | | |
| **ASP** (M1, 45°C) | HMo | HMo | HMo | HMo | | |

**Tableau 4**

| | (F'1) | (F'2) | (F'3) | (F'4) | (F'5) | (F'6) | (F'7) |
|---|---|---|---|---|---|---|---|
| **ASP** (J1, 20°C) | HNMo | Het-NMo | Het-NMo | Het-NMo | HNMo | HNMo | HNMo |
| **ASP** (J7, 20°C) | HNMo | Het-NMo | Het-NMo | Het-NMo | HNMo | HNMo | HNMo |
| **µ** (J7) | 98000 | 98000 | n.m | 152 000 | 66 000 | n.m | n.m |
| **ASP** (M1, 20°C) | HNMo | Het-NMO | Het-NMo | Het-NMo | HNMo | HNMo | HNMo |
| **µ** (M1) | 102 000 | 97 000 | n.m | 220 000 | n.m | n.m | n.m |
| **ASP** (J7, 45°C) | HNMo | Het-NMO | Het-NMo | Het-NMo | n.m | n.m | n.m |
| **ASP** (M1, 45°C) | HNMo | Het-NMO | Het-NMo | Het-NMo | n.m | n.m | n.m |

### II-2.3 Analyse des résultats

Les émulsions huile-dans-eau (F₁), (F₂), (F₃), (F₄), (F₅), (F₆), (F₇), (F₈), (F₉) et (F₁₀) selor l'invention, se caractérisent :
- Par un aspect homogène et une forme de mousse après une durée d'un jour, de 7 jours et d'un mois de stockage à une température de 20°C,
- Par un aspect homogène et une forme de mousse après une durée de 7 jours et d'un mois de stockage à une température de 45°C,
- Par une stabilité satisfaisante des valeurs de viscosités dynamiques mesurées après 1 mois de stockage à 20°C, en comparaison avec les valeurs de viscosités dynamiques mesurées après 7 jours de stockage à 20°C, lesdites valeurs de viscosité dynamique étant mesurées au moyen d'un viscosimètre de modèle Brookfield RV à 20°C à une vitesse de cinq tours par minute.

L'émulsion comparative (F'₁), se distinguant des émulsions (F2), (F4), (F6) et (F7) selon l'invention par une valeur du ratio massique (Ei)/(P) égale à 5/1 ((F2), (F4), (F6) et (F7) se caractérisant par des valeurs du ratio massique (Ei)/(P) respectivement de 10/1, de 8/1, de 12/1 et de 15/1), montre un aspect homogène et une absence de forme mousse alors que les émulsions (F2), (F4), (F6) et (F7) selon l'invention montrent un aspect homogène et une forme mousse.

L'émulsion comparative (F'₂), comprenant comme système polymérique un polyélectrolyte anionique réticulé, et se caractérisant par un ratio massique [épaississant inorganique (Ei)/polyélectrolyte anionique réticulé] égal à 10/1, présente un aspect hétérogène (présence de grumeaux) et ne se trouve pas sous la forme d'une mousse.

Les émulsions comparatives (F'₃) et (F'₄), comprenant comme système polymérique un polyélectrolyte anionique réticulé, dont le squelette polymérique comprend un monomère à chaîne alkyle non éthoxylé, et se caractérisant par des ratios massique [épaississant inorganique (Ei)/polyélectrolyte anionique réticulé] respectivement égaux à 10/1 et à 20/1, présentent un aspect hétérogène (présence de grumeaux) et ne se trouvent pas sous la forme d'une mousse.

L'émulsion comparative (F'₅), comprenant comme système polymérique le polyélectrolyte anionique réticulé (P) et ne comprenant pas d'épaississant inorganique (Ei), montre un aspect homogène et une absence de forme mousse alors que les émulsions (F2), (F4), (F6) et (F7) selon l'invention montrent un aspect homogène et une forme mousse.

Les émulsions comparatives (F'₆) et (F'₇), comprenant comme système polymérique le polyélectrolyte anionique réticulé (P) et ne comprenant pas d'épaississant inorganique (Ei), et dont le procédé de préparation comprend une étape de barbotage d'azote, montrent un aspect homogène et une absence de forme mousse, alors que les émulsions (F2), (F4), (F6) et (F7) selon l'invention montrent un aspect homogène et une forme mousse.

## Revendications

1. Emulsion (E₁) de type huile-dans-eau se présentant sous la forme d'une mousse, caractérisée en ce qu'elle comprend pour 100% de sa masse :
a) - De 27% à 89,9% massique d'une phase aqueuse (A₁) cosmétiquement acceptable ;
b) - De 0,1% à 3% massique d'un polyélectrolyte anionique réticulé (P) issu de la polymérisation, en présence d'au moins un agent de réticulation, de l'acide 2-méthyl 2-[(1-oxo 2-propènyl) amino] 1-propanesulfonique partiellement ou totalement salifié, avec au moins un monomère neutre choisi parmi l'acrylamide, l'acrylate de (2-hydroxy éthyle), les N,N-dialkyl acrylamides, dans lesquels chacun des groupes alkyle comportent entre un et quatre atomes de carbones, et au moins un monomère de formule (I) : 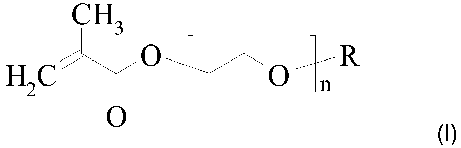 dans laquelle R représente un radical alkyle linéaire ou ramifié comportant de huit à vingt atomes de carbone et n représente un nombre entier supérieur ou égal à un et inférieur ou égal à vingt ;
c) - De 5% à 20% massique d'au moins un agent épaississant inorganique (Ei) choisi parmi les phyllosilicates naturels ou synthétiques, modifiés ou non modifiés ;
d) - De 5% à 50% massique d'une phase grasse (A₂) comprenant pour 100% de sa masse :
d₁) - De 0,2% à 25% massique d'un système émulsionnant (S) comprenant un ou plusieurs tensioactifs émulsionnants sélectionnés parmi les compositions d'alkylpolyglycosides ; les compositions d'alkylpolyglycosides et d'alcools gras ; les acides gras polyéthoxylés comportant de 10 à 40 atomes de carbone et ayant un degré d'éthoxylation compris entre 5 et 100 ; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone ; les alcools gras polyéthoxylés comportant de 10 à 40 atomes de carbone et ayant un degré d'éthoxylation compris entre 2 et 100 ; les compositions d'alcools gras polyéthoxylés comportant de 10 à 40 atomes de carbone et ayant un degré d'éthoxylation compris entre 2 et 100 et d'alcools gras comportant de 12 à 40 atomes de carbone; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone et d'alcools gras comportant de 12 à 40 atomes de carbone ; et
d₂) - De 75% à 99,8% massique d'au moins une huile et optionnellement d'au moins une cire ;
ladite émulsion (E₁) étant en outre **caractérisée en ce que** le rapport massique entre l'agent épaississant inorganique (Ei) et le polyélectrolyte anionique réticulé (P) est supérieur ou égal à 6/1 et inférieur ou égal à 20/1.

2. Emulsion (E₁) telle que définie à la revendication 1, caractérisée en ce qu'elle comprend pour 100% de sa masse :
d) - De 5% à 50% massique d'une phase grasse (A₃) comprenant pour 100% de sa masse :
d₁) - De 0,2% à 20% massique dudit système émulsionnant (S) ; et
d₂) - De 80% à 99,8% massique d'au moins une huile et optionnellement d'au moins une cire.

3. Emulsion (E₁) telle que définie à la revendication 1, caractérisée en ce qu'elle comprend pour 100% de sa masse :
a) - De 27% à 84,5% massique De ladite phase aqueuse (A₁) ;
b) - De 0,5% à 3% massique dudit polyélectrolyte anionique réticulé (P).

4. Emulsion (E₁) telle que définie à l'une quelconque des revendications 1, 2 ou 3, **caractérisée en ce que** ledit polyélectrolyte anionique réticulé (P) comporte, pour 100% molaire de ses monomères constitutifs :
- De 20% molaire à 80% molaire d'unités monomériques issues dudit acide 2-méthyl 2-[(1-oxo 2-propènyl) amino] 1-propanesulfonique partiellement ou totalement salifiée ;
- De 15% molaire à 75% molaire d'unités monomériques issues d'au moins un monomère neutre choisi parmi l'acrylamide, l'acrylate de (2-hydroxy éthyle), les N,N-dialkyl acrylamides, dans lesquels chacun des groupes alkyle comportent entre un et quatre atomes de carbones,
- De 0,5% à 5% molaire d'unités monomériques issues dudit monomère de formule (I) telle que définie précédemment.

5. Emulsion (E₁) telle que définie à l'une ou quelconque des revendications 1 à 4, **caractérisée en ce que** ledit monomère neutre est choisi parmi l'acrylamide, l'acrylate de (2-hydroxy éthyle) ou le N,N-diméthyl acrylamide.

6. Emulsion (E₁) telle que définie à l'une ou quelconque des revendications 1 à 5, **caractérisée en ce que** ledit monomère de formule (I) est le méthacrylate de lauryle tétraéthoxylé.

7. Emulsion (E₁) telle que définie à la revendication 6, **caractérisée en ce que** ledit polyélectrolyte anionique réticulé (P) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

8. Emulsion (E₁) telle que définie à l'une ou quelconque des revendications 1 à 7, **caractérisée en ce que** ledit polyélectrolyte anionique réticulé (P) comporte pour 100% molaire :
- De 60% molaire à 80% molaire d'unités monomériques issues de l'acide 2-méthyl 2-[(1-oxo 2-propènyl) amino] 1-propanesulfonique partiellement salifié sous forme d'ammonium,
- De 15% molaire à 39,5% molaire d'unités monomériques issues du N,N-diméthyl acrylamide, et
- De 0,5% molaire à 5% molaire d'unités monomériques issues du méthacrylate de lauryle tétraéthoxylé.

9. Emulsion (E₁) telle que définie à l'une ou quelconque des revendications 1 à 8, **caractérisée en ce que** l'épaississant inorganique (Ei) est choisi parmi la kaolinite, la montmorillonite, la saponite, la bentonite ou l'hectorite.

10. Emulsion (E₁) telle que définie à l'une ou quelconque des revendications 1 à 9, **caractérisée en ce que** le rapport massique entre ledit agent épaississant inorganique (Ei) et le dit polyélectrolyte anionique réticulé (P) est supérieur ou égal à 8/1.

11. Emulsion (E₁) telle que définie à l'une ou quelconque des revendications 1 à 10, caractérisée en ce qu'elle comprend en outre, pour 100% de sa masse,
e) - une proportion massique supérieure ou égale à 0,01% et inférieure ou égale à 0,5% d'au moins un polysaccharide (PS) choisi parmi les polysaccharides constitués de dérivés d'oses et par les polysaccharides constitués uniquement d'oses.

12. Emulsion (E₁) telle que définie à la revendication 11, **caractérisée en ce que** le polysaccharide (PS) est choisi parmi le groupe constitué par les carraghénanes, l'agar, l'exsudat de la gomme arabique, l'exsudat de la gomme de karaya, la gomme xanthane, la gomme gellane, le chitosane, les glucoanes, les galactomannanes provenant de la gomme de cassia, les galactomannanes provenant de la gomme de caroube, les galactomannanes provenant de la gomme de tara, les galactomannanes provenant de la gomme de guar et les galactomannanes provenant de la gomme de fenugrec

13. Emulsion (E₁) telle que définie à l'une quelconque des revendications 11 ou 12, caractérisée en ce qu'elle comprend pour 100% de sa masse :
a) - De 52,5% à 76,4% massique d'une phase aqueuse (A₁) cosmétiquement acceptable ;
b) - De 0,5% à 2% massique d'un polyélectrolyte anionique réticulé (P) issu de la polymérisation, en présence d'au moins un agent de réticulation, de 60% à 80% molaire d'unités monomériques issues de l'acide 2-méthyl 2-[(1-oxo 2-propènyl) amino] 1-propanesulfonique partiellement ou totalement salifié sous forme de sel d'ammonium, avec de 15% molaire à 39,5% molaire d'unités monomériques issues du N,N-diméthyl acrylamide, et de 0,5% à 5% molaire d'unités monomériques issues du méthacrylate de lauryle tétraéthoxylé,
c) - De 8% à 15% massique d'au moins un agent épaississant inorganique (Ei) choisi parmi les parmi la kaolinite, la montmorillonite, la saponite, la bentonite ou l'hectorite.
d) - De 15% à 30% massique d'une phase grasse (A₂) comprenant pour 100% de sa masse :
d₁) - De 5% à 25% massique d'un système émulsionnant (S) comprenant un ou plusieurs tensioactifs émulsionnants sélectionnés parmi les compositions d'alkylpolyglycosides ; les compositions d'alkylpolyglycosides et d'alcools gras ; les acides gras polyéthoxylés comportant de 10 à 40 atomes de carbone et ayant un degré d'éthoxylation compris entre 5 et 100 ; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone ; les alcools gras polyéthoxylés comportant de 10 à 40 atomes de carbone et ayant un degré d'éthoxylation compris entre 2 et 100 ; les compositions d'alcools gras polyéthoxylés comportant de 10 à 40 atomes de carbone et ayant un degré d'éthoxylation compris entre 2 et 100 et d'alcools gras comportant de 12 à 40 atomes de carbone; les compositions d'acides gras polyéthoxylés, comportant de 10 à 40 atomes de carbone et ayant un degré d'éthoxylation compris entre 5 et 100, et d'esters de glycérol d'acides gras comportant de 10 à 40 atomes de carbone et d'alcools gras comportant de 12 à 40 atomes de carbone ; et
d₂) - De 75% à 95% massique d'au moins une huile et optionnellement d'au moins une cire ;
e) - De 0,1% à 0,5% massique d'au moins un polysaccharides choisi parmi l'agar, l'exsudat de la gomme arabique, l'exsudat de la gomme de karaya, la gomme xanthane, la gomme gellane, les galactomannanes provenant de la gomme de cassia, les galactomannanes provenant de la gomme de caroube, les galactomannanes provenant de la gomme de tara, les galactomannanes provenant de la gomme de guar et les galactomannanes provenant de la gomme de fenugrec,
ladite émulsion (E₁) étant en outre **caractérisée en ce que** le rapport massique entre l'agent épaississant inorganique (Ei) et le polyélectrolyte anionique réticulé (P) est supérieur ou égal à 8/1 et inférieur ou égal à 20/1.

14. Procédé de préparation d'une émulsion (E₁) de type huile-dans-eau se présentant sous la forme d'une mousse et telle que définie à l'une ou quelconque des revendications 1 à 13, comprenant :
Au moins une étape a) de préparation d'une phase (A') par mélange du polyélectrolyte anionique réticulé (P), et optionnellement du polysaccharide (PS), dans la phase grasse (A₂) ; et
Au moins une étape b) d'émulsification de ladite phase (A') obtenue à l'issue de l'étape a) avec la phase aqueuse (A₁) cosmétiquement acceptable pour obtenir une émulsion (E'₁) ;et
Au moins une étape c) d'obtention de l'émulsion (E₁) par mélange de l'agent épaississant inorganique (Ei) dans l'émulsion (E'₁) obtenue à l'issue de l'étape b)

15. Utilisation de l'émulsion (E₁) telle que définie à l'une ou quelconque des revendications 1 à 13, pour le traitement cosmétique de la peau, des cheveux, du cuir chevelu, des muqueuses et/ou les ongles.

16. Utilisation de l'émulsion (E₁) telle que définie à la revendication 15, pour le nettoyage et/ou pour le soin de la peau, des cheveux, du cuir chevelu, des muqueuses et/ou des ongles, et pour le maquillage de la peau, des cheveux et/ou des ongles.

## Patentansprüche

1. Emulsion (E₁) des Typs Öl-in-Wasser, die in der Form eines Schaums vorliegt, **dadurch gekennzeichnet, dass** sie für 100 % ihrer Masse Folgendes umfasst:
a) - von 27 Massen-% bis 89,9 Massen-% eine kosmetisch annehmbare wässrige Phase (A₁);
b) - von 0,1 Massen-% bis 3 Massen-% einen vernetzten anionischen Polyelektrolyt (P), der aus der Polymerisation von teilweise oder vollständig versalzter 2-Methyl-2-[(1-oxo-2-propenyl)amino]-1-propansulfonsäure mit mindestens einem neutralen Monomer, gewählt aus Acrylamid, (2-Hydroxyethyl)acrylat, N,N-Dialkylacrylamiden, in Gegenwart von mindestens einem Vernetzungsmittel, wobei jede der Alkylgruppen zwischen einem und vier Kohlenstoffatomen umfasst, und mindestens einem Monomer der Formel (I) stammt: 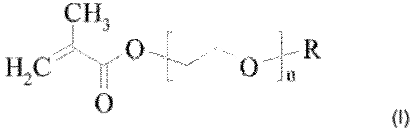 wobei R einen linearen oder verzweigten, von acht bis zwanzig Kohlenstoffatome umfassenden, Alkylrest darstellt und n eine ganze Zahl größer als oder gleich eins und kleiner als oder gleich zwanzig darstellt;
c) - von 5 Massen-% bis 20 Massen-% mindestens ein anorganisches Verdickungsmittel (Ei), gewählt aus natürlichen oder synthetischen, modifizierten oder nicht modifizierten Phyllosilikaten;
d) - von 5 Massen-% bis 50 Massen-% eine fette Phase (A₂), die für 100 % ihrer Masse Folgendes umfasst:
d₁) - von 0,2 Massen-% bis 25 Massen-% ein Emulgatorsystem (S), umfassend ein oder mehrere emulgierende Tenside, ausgewählt aus Zusammensetzungen von Alkylpolyglycosiden; Zusammensetzungen von Alkylpolyglycosiden und Fettalkoholen; polyethoxylierten Fettsäuren, umfassend von 10 bis 40 Kohlenstoffatome und einen Ethoxylierungsgrad im Bereich zwischen 5 und 100 aufweisend; Zusammensetzungen von polyethoxylierten Fettsäuren, umfassend von 10 bis 40 Kohlenstoffatome und einen Ethoxylierungsgrad im Bereich zwischen 5 und 100 aufweisend, und Glycerolestern von Fettsäuren, umfassend von 10 bis 40 Kohlenstoffatome; polyethoxylierten Fettsäuren, umfassend von 10 bis 40 Kohlenstoffatome und einen Ethoxylierungsgrad im Bereich zwischen 2 und 100 aufweisend; Zusammensetzungen von polyethoxylierten Fettalkoholen, umfassend von 10 bis 40 Kohlenstoffatomen und einen Ethoxylierungsgrad im Bereich zwischen 2 und 100 aufweisend und Fettalkoholen, umfassend von 12 bis 40 Kohlenstoffatome; Zusammensetzungen von polyethoxylierten Fettsäuren, umfassend von 10 bis 40 Kohlenstoffatome und einen Ethoxylierungsgrad im Bereich zwischen 5 und 100 aufweisend, und Glycerolestern von Fettsäuren, umfassend von 10 bis 40 Kohlenstoffatome, und Fettalkoholen, umfassend von 12 bis 40 Kohlenstoffatome; und
d₂) - von 75 Massen-% bis 99,8 Massen-% mindestens ein Öl und wahlweise mindestens ein Wachs;
wobei die Emulsion (E₁) des Weiteren **dadurch gekennzeichnet ist, dass** das Massenverhältnis zwischen dem anorganischen Verdickungsmittel (Ei) und dem anionischen vernetzten Polyelektrolyt (P) größer als oder gleich 6/1 und kleiner als oder gleich 20/1 ist.

2. Emulsion (E₁) nach Anspruch 1, **dadurch gekennzeichnet, dass** sie für 100 % ihrer Masse Folgendes umfasst:
d) - von 5 Massen-% bis 50 Massen-% eine fette Phase (A₃), die für 100 % ihrer Masse Folgendes umfasst:
d₁) - von 0,2 Massen-% bis 20 Massen-% das Emulgatorsystem (S); und
d₂) - von 80 Massen-% bis 99,8 Massen-% mindestens ein Öl und wahlweise mindestens ein Wachs.

3. Emulsion (E₁) nach Anspruch 1, **dadurch gekennzeichnet, dass** sie für 100 % ihrer Masse Folgendes umfasst:
a) - von 27 Massen-% bis 84,5 Massen-% die wässrige Phase (A₁);
b) - von 0,5 Massen-% bis 3 Massen-% den vernetzten anionischen Polyelektrolyt (P).

4. Emulsion (E₁) nach einem der vorstehenden Ansprüche 1, 2 oder 3, **dadurch gekennzeichnet, dass** der vernetzte anionische Polyelektrolyt (P) für 100 Mol-% seiner konstituierenden Monomere Folgendes umfasst:
- von 20 Mol-% bis 80 Mol-% aus der teilweise oder vollständig versalzten 2-Methyl-2-[(1-oxo-2-propenyl)-amino]-1-propansulfonsäure stammende Monomereinheiten;
- von 15 Mol-% bis 75 Mol-% von mindestens aus einem neutralen Monomer, gewählt aus Acrylamid, (2-Hydroxyethyl)acrylat, N,N-Dialkylacrylamiden stammende Monomereinheiten, wobei jede der Alkylgruppen zwischen einem und vier Kohlenstoffatome umfasst,
- von 0,5 Mol-% bis 5 Mol-% aus dem Monomer der Formel (I) stammende Monomereinheiten, wie vorher definiert.

5. Emulsion (E₁) nach einem der Ansprüche 1 bis 4, **dadurch gekennzeichnet, dass** das neutrale Monomer gewählt ist aus Acrylamid, (2-Hydroxyethyl)acrylat oder N,N-Dimethylacrylamid.

6. Emulsion (E₁) nach einem der Ansprüche 1 bis 5, **dadurch gekennzeichnet, dass** das Monomer der Formel (I) tetraethoxyliertes Laurylmethacrylat ist.

7. Emulsion (E₁) nach Anspruch 6, **dadurch gekennzeichnet, dass** der vernetzte anionische Polyelektrolyt (P) ein mit Trimethylolpropantriacrylat vernetztes Terpolymer der teilweise oder vollständig versalzten 2-Methyl-2-[(1-oxo-2-propenyl)-amino]-1-propansulfonsäure in der Form des Ammoniumsalzes, des N,N-Dimethylacrylamids und des tetraethoxylierten Laurylmethacrylats ist.

8. Emulsion (E₁) nach einem der Ansprüche 1 bis 7, **dadurch gekennzeichnet, dass** der vernetzte anionische Polyelektrolyt (P) für 100 Mol-% Folgendes umfasst:
- von 60 Mol-% bis 80 Mol-% aus der teilweise in Ammoniumform versalzten 2-Methyl-2-[(1-oxo-2-propenyl)-amino]-1-propansulfonsäure stammende Monomereinheiten;
- von 15 Mol-% bis 39,5 Mol-% aus N,N-Dimethylacrylamid stammende Monomereinheiten, und
- von 0,5 Mol-% bis 5 Mol-% aus tetraethoxyliertem Laurylmethacrylat stammende Monomereinheiten.

9. Emulsion (E₁) nach einem der Ansprüche 1 bis 8, **dadurch gekennzeichnet, dass** das anorganische Verdickungsmittel (Ei) gewählt ist aus Kaolinit, Montmorillonit, Saponit, Bentonit oder Hectorit.

10. Emulsion (E₁) nach einem der Ansprüche 1 bis 9, **dadurch gekennzeichnet, dass** das Massenverhältnis zwischen dem anorganischen Verdickungsmittel (Ei) und dem vernetzten anionischen Polyelektrolyt (P) größer als oder gleich 8/1 ist.

11. Emulsion (E₁) nach einem der Ansprüche 1 bis 10, **dadurch gekennzeichnet, dass** sie des Weiteren für 100 % ihrer Masse Folgendes umfasst,
e) - einen Massenanteil größer als oder gleich 0,01% und kleiner als oder gleich 0,5 % von mindestens einem Polysaccharid (PS), gewählt aus Polysacchariden, bestehend aus Derivaten von Monosacchariden und Polysacchariden, die ausschließlich aus Monosacchariden bestehen.

12. Emulsion (E₁) nach Anspruch 11, **dadurch gekennzeichnet, dass** das Polysaccharid (PS) gewählt ist aus der Gruppe, bestehend aus Carrageenanen, Agar, Exsudat von Gummi Arabicum, Exsudat von Karayagummi, Xanthangummi, Gellangummi, Chitosan, Glucoanen, von Cassiagummi herrührenden Galaktomannanen, von Johannisbrotgummi herrührenden Galaktomannanen, von Taragummi herrührenden Galaktomannanen, von Guargummi herrührenden Galaktomannanen und von Bockshornkleegummi herrührenden Galaktomannanen.

13. Emulsion (E₁) nach einem der Ansprüche 11 oder 12, **dadurch gekennzeichnet, dass** sie für 100 % ihrer Masse Folgendes umfasst:
a) - von 52,5 Massen-% bis 76,4 Massen-% eine kosmetisch annehmbare wässrige Phase (A₁);
b) - von 0,5 Massen-% bis 2 Massen-% einen aus der Polymerisation von 60 Mol-% bis 80 Mol-% von aus in Ammoniumform teilweise oder vollständig versalzter 2-Methyl-2-[(1-oxo-2-propenyl)-amino]-1-propansulfonsäure stammenden Monomereinheiten mit von 15 Mol-% bis 39,5 Mol-% von aus N,N-Dimethylacrylamid stammenden Monomereinheiten und von 0,5 Mol-% bis 5 Mol-% von aus tetraethoxyliertem Laurylmethacrylat stammenden Monomereinheiten in Gegenwart von mindestens einem Vernetzungsmittel vernetzten anionischen Polyelektrolyt (P),
c) - von 8 Massen-% bis 15 Massen-% mindesten ein anorganisches Verdickungsmittel (Ei), gewählt aus Kaolinit, Montmorillonit, Saponit, Bentonit oder Hectorit.
d) - von 15 Massen-% bis 30 Massen-% eine fette Phase (A₂), die für 100 % ihrer Masse Folgendes umfasst:
d₁) - von 5 Massen-% bis 25 Massen-% ein Emulgatorsystem (S), umfassend ein oder mehrere emulgierende Tenside, ausgewählt aus Zusammensetzungen von Alkylpolyglycosiden; Zusammensetzungen von Alkylpolyglycosiden und Fettalkoholen; polyethoxylierten Fettsäuren, umfassend von 10 bis 40 Kohlenstoffatome und einen Ethoxylierungsgrad im Bereich zwischen 5 und 100 aufweisend; Zusammensetzungen von polyethoxylierten Fettsäuren, umfassend von 10 bis 40 Kohlenstoffatome und einen Ethoxylierungsgrad im Bereich zwischen 5 und 100 aufweisend, und Glycerolestern von Fettsäuren, umfassend von 10 bis 40 Kohlenstoffatome; polyethoxylierten Fettsäuren, umfassend von 10 bis 40 Kohlenstoffatome und einen Ethoxylierungsgrad im Bereich zwischen 2 und 100 aufweisend; Zusammensetzungen von polyethoxylierten Fettalkoholen, umfassend von 10 bis 40 Kohlenstoffatome und einen Ethoxylierungsgrad im Bereich zwischen 2 und 100 aufweisend und Fettalkoholen, umfassend von 12 bis 40 Kohlenstoffatome; Zusammensetzungen von polyethoxylierten Fettsäuren, umfassend von 10 bis 40 Kohlenstoffatome und einen Ethoxylierungsgrad im Bereich zwischen 5 und 100 aufweisend, und Glycerolestern von Fettsäuren, umfassend von 10 bis 40 Kohlenstoffatome, und Fettalkoholen, umfassend von 12 bis 40 Kohlenstoffatome; und
d₂) - von 75 Massen-% bis 95 Massen-% mindestens ein Öl und wahlweise mindestens ein Wachs;
e) - von 0,1 Massen-% bis 0,5 Massen-% mindestens ein Polysaccharid, gewählt aus Agar, Exsudat von Gummi Arabicum, Exsudat von Karayagummi, Xanthangummi, Gellangummi, von Cassiagummi herrührenden Galaktomannanen, von Johannisbrotgummi herrührenden Galaktomannanen, von Taragummi herrührenden Galaktomannanen, von Guargummi herrührenden Galaktomannanen und von Bockshornkleegummi herrührenden Galaktomannanen,
wobei die Emulsion (E₁) weiter **dadurch gekennzeichnet ist, dass** das Massenverhältnis zwischen dem anorganischen Verdickungsmittel (Ei) und dem anionischen vernetzten Polyelektrolyt (P) größer als oder gleich 8/1 und kleiner als oder gleich 20/1 ist.

14. Zubereitungsverfahren einer Emulsion (E₁) der Art Öl-in-Wasser, die in der Form eines Schaums vorliegt und nach einem der Ansprüche 1 bis 13, umfassend:
mindestens einen Schritt a) der Zubereitung einer Phase (A') durch Mischen von vernetztem anionischen Polyelektrolyt (P) und wahlweise von Polysaccharid (PS) in die fette Phase (A₂); und
mindestens einen Schritt b) des Emulgierens der nach Abschluss von Schritt a) erhaltenen Phase (A') mit der kosmetisch annehmbaren wässrigen Phase (A₁), um eine Emulsion (E'₁) zu erhalten; und
mindestens einen Schritt c) des Erhaltens der Emulsion (E₁) durch Mischen des anorganischen Verdickungsmittels (Ei) in die nach Abschluss des Schritts b) erhaltene Emulsion (E₁)

15. Verwendung der Emulsion (E₁) nach einem der Ansprüche 1 bis 13 für die kosmetische Behandlung der Haut, der Haare, der Kopfhaut, der Schleimhäute und/oder der Nägel.

16. Verwendung der Emulsion (E₁) nach Anspruch 15 für die Reinigung und/oder die Pflege der Haut, der Haare, der Kopfhaut, der Schleimhäute und/oder der Nägel und zum Schminken der Haut, der Haare und/oder der Nägel.

## Claims

1. Oil-in-water emulsion (E₁) in the form of a foam, **characterised in that** it comprises, per 100% of its weight:
a) - from 27% to 89.9% by weight of a cosmetically acceptable aqueous phase (A1);
b) - from 0.1% to 3% by weight of a crosslinked anionic polyelectrolyte (P) resulting from the polymerisation, in the presence of at least one crosslinking agent, of partially or totally salified 2-methyl-2-[(l-oxo-2-propenyl)amino]-1-propanesulfonic acid with at least one neutral monomer selected from acrylamide, (2-hydroxyethyl) acrylate, N,N-dialkylacrylamides, in which each of the alkyl groups comprises between one and four carbon atoms, and at least one monomer of formula (I): 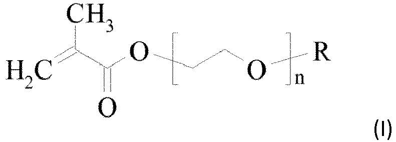 in which R represents a linear or branched alkyl radical comprising from eight to twenty carbon atoms and n represents an integer greater than or equal to one and less than or equal to twenty;
c) - from 5% to 20% by weight of at least one inorganic thickening agent (Ei) chosen from natural or synthetic, modified or unmodified phyllosilicates;
d) - from 5% to 50% by weight of a fatty phase (A₂) comprising, per 100% of its weight:
d₁) - from 0.2% to 25% by weight of an emulsifying system (S) comprising one or more emulsifying surfactants selected from alkylpolyglycoside compositions; alkylpolyglycoside and fatty alcohol compositions; polyethoxylated fatty acids comprising from 10 to 40 carbon atoms and having a degree of ethoxylation of between 5 and 100; compositions of polyethoxylated fatty acids comprising from 10 to 40 carbon atoms and having a degree of ethoxylation of between 5 and 100, and glycerol esters of fatty acids comprising from 10 to 40 carbon atoms; polyethoxylated fatty alcohols comprising from 10 to 40 carbon atoms and having a degree of ethoxylation of between 2 and 100; compositions of polyethoxylated fatty alcohols comprising from 10 to 40 carbon atoms and having a degree of ethoxylation of between 2 and 100 and fatty alcohols comprising from 12 to 40 carbon atoms; compositions of polyethoxylated fatty acids comprising from 10 to 40 carbon atoms and having a degree of ethoxylation of between 5 and 100 and glycerol esters of fatty acids comprising from 10 to 40 carbon atoms and fatty alcohols comprising from 12 to 40 carbon atoms; and
d₂) - from 75% to 99.8% by weight of at least one oil and optionally at least one wax;
said emulsion (E₁) being further **characterised in that** the weight ratio between the inorganic thickening agent (Ei) and the crosslinked anionic polyelectrolyte (P) is greater than or equal to 6/1 and less than or equal to 20/1.

2. Emulsion (E₁) as defined in claim 1, **characterised in that** it comprises, per 100% of its weight:
d) - from 5% to 50% by weight of a fatty phase (A₃) comprising, per 100% of its weight:
d₁) - from 0.2% to 20% by weight of said emulsifying system (S); and
d₂) - from 80% to 99.8% by weight of at least one oil and optionally at least one wax.

3. Emulsion (E₁) as defined in claim 1, **characterised in that** it comprises, per 100% of its weight:
a) - from 27% to 84.5% by weight of said aqueous phase (A₁);
b) - from 0.5% to 3% by weight of said crosslinked anionic polyelectrolyte (P).

4. Emulsion (E₁) as defined in any of claims 1, 2 or 3, **characterised in that** said crosslinked anionic polyelectrolyte (P) comprises, per 100 mol% of its constituent monomers:
- from 20 mol% to 80 mol% of monomer units derived from said partially or totally salified 2-methyl-2-[(1-oxo-2-propenyl)amino]-1-propanesulfonic acid;
- from 15 mol% to 75 mol% of monomer units derived from at least one neutral monomer chosen from acrylamide, (2-hydroxyethyl) acrylate, N,N-dialkylacrylamides, in which each of the alkyl groups comprises between one and four carbon atoms,
- from 0.5 mol% to 5 mol% of monomer units derived from said monomer of formula (I) as defined above.

5. Emulsion (E₁) as defined in any of claims 1 to 4, **characterised in that** said neutral monomer is selected from acrylamide, (2-hydroxyethyl) acrylate or N,N-dimethylacrylamide.

6. Emulsion (E₁) as defined in any of claims 1 to 5, **characterised in that** said monomer of formula (I) is tetraethoxylated lauryl methacrylate.

7. Emulsion (E₁) as defined in claim 6, **characterised in that** said crosslinked anionic polyelectrolyte (P) is a terpolymer of 2-methyl-2-[(1-oxo-2-propenyl)amino]-1-propanesulfonic acid, partially salified in ammonium salt form, of N,N-dimethylacrylamide and of tetraethoxylated lauryl methacrylate, crosslinked with trimethylolpropane triacrylate.

8. Emulsion (E₁) as defined in any of claims 1 to 7, **characterised in that** said crosslinked anionic polyelectrolyte (P) comprises, per 100 mol%:
- from 60 mol% to 80 mol% of monomer units derived from 2-methyl-2-[(1-oxo-2-propenyl)amino]-1-propanesulfonic acid, partially salified in ammonium form,
- from 15 mol% to 39.5 mol% of monomer units derived from N,N-dimethylacrylamide, and
- from 0.5 mol% to 5 mol% of monomer units derived from tetraethoxylated lauryl methacrylate.

9. Emulsion (E₁) as defined in any of claims 1 to 8, **characterised in that** the inorganic thickener (Ei) is chosen from kaolinite, montmorillonite, saponite, bentonite or hectorite.

10. Emulsion (E₁) as defined in any of claims 1 to 9, **characterised in that** the weight ratio between said inorganic thickening agent (Ei) and said crosslinked anionic polyelectrolyte (P) is greater than or equal to 8/1.

11. Emulsion (E₁) as defined in any of claims 1 to 10, **characterised in that** it also comprises, per 100% of its weight,
(e) - a proportion by weight greater than or equal to 0.01% and less than or equal to 0.5% of at least one polysaccharide (PS) selected from polysaccharides consisting of ose derivatives and polysaccharides consisting solely of oses.

12. Emulsion (E₁) as defined in claim 11, **characterised in that** the polysaccharide (PS) is selected from the group consisting of carrageenans, agar, exudate of gum arabic, exudate of gum karaya, xanthan gum, gellan gum, chitosan, glucans, galactomannans originating from cassia gum, galactomannans originating from locust bean gum, galactomannans originating from tara gum, galactomannans originating from guar gum and galactomannans originating from fenugreek gum.

13. Emulsion (E₁) as defined in claim 11 or 12, **characterised in that** it comprises, per 100% of its weight:
a) - from 52.5% to 76.4% by weight of a cosmetically acceptable aqueous phase (A1);
b) - from 0.5% to 2% by weight of a crosslinked anionic polyelectrolyte (P) resulting from the polymerisation, in the presence of at least one crosslinking agent, of 60 mol% to 80 mol% of monomer units derived from 2-methyl-2-[(1-oxo-2-propenyl)amino]-1-propanesulfonic acid, partially or totally salified in ammonium salt form, with 15 mol% to 39.5 mol% of monomer units derived from N,N-dimethylacrylamide, and 0.5 mol% to 5 mol% of monomer units derived from tetraethoxylated lauryl methacrylate,
c) - from 8% to 15% by weight of at least one inorganic thickening agent (Ei) selected from kaolinite, montmorillonite, saponite, bentonite or hectorite,
d) - from 15% to 30% by weight of a fatty phase (A₂) comprising, per 100% of its weight:
d₁) -from 5% to 25% by weight of an emulsifying system (S) comprising one or more emulsifying surfactants selected from alkylpolyglycoside compositions; alkylpolyglycoside and fatty alcohol compositions; polyethoxylated fatty acids comprising from 10 to 40 carbon atoms and having a degree of ethoxylation of between 5 and 100; compositions of polyethoxylated fatty acids comprising from 10 to 40 carbon atoms and having a degree of ethoxylation of between 5 and 100, and glycerol esters of fatty acids comprising from 10 to 40 carbon atoms; polyethoxylated fatty alcohols comprising from 10 to 40 carbon atoms and having a degree of ethoxylation of between 2 and 100;
compositions of polyethoxylated fatty alcohols comprising from 10 to 40 carbon atoms and having a degree of ethoxylation of between 2 and 100 and fatty alcohols comprising from 12 to 40 carbon atoms; compositions of polyethoxylated fatty acids comprising from 10 to 40 carbon atoms and having a degree of ethoxylation of between 5 and 100, and glycerol esters of fatty acids comprising from 10 to 40 carbon atoms and fatty alcohols comprising from 12 to 40 carbon atoms; and
d₂) - from 75% to 95% by weight of at least one oil and optionally at least one wax;
e) - from 0.1% to 0.5% by weight of at least one polysaccharide selected from agar, exudate of gum arabic, exudate of gum karaya, xanthan gum, gellan gum, galactomannans originating from cassia gum, galactomannans originating from locust bean gum, galactomannans originating from tara gum, galactomannans originating from guar gum and galactomannans originating from fenugreek gum,
said emulsion (E1) being **characterised in that** the weight ratio between the inorganic thickening agent (Ei) and the crosslinked anionic polyelectrolyte (P) is greater than or equal to 8/1 and less than or equal to 20/1.

14. Process for preparing an oil-in-water emulsion (E₁) in the form of a foam and as defined in any of claims 1 to 13, comprising:
at least one step a) of preparing a phase (A') by mixing the crosslinked anionic polyelectrolyte (P), and optionally the polysaccharide (PS), in the fatty phase (A₂); and
at least one step b) of emulsifying said phase (A') obtained at the end of step a) with the cosmetically acceptable aqueous phase (A₁) in order to obtain an emulsion (E'₁); and
at least one step c) of obtaining the emulsion (E₁) by mixing the inorganic thickening agent (Ei) in the emulsion (E'₁) obtained at the end of step b).

15. Use of the emulsion (E₁) as defined in any of claims 1 to 13 for cosmetic treatment of the skin, hair, scalp, mucous membranes and/or nails.

16. Use of the emulsion (E₁) as defined in claim 15 for cleansing and/or caring for the skin, hair, scalp, mucous membranes and/or nails, and for makeup of the skin, hair and/or nails.
